# EUROPEAN PATENT APPLICATION

(11) **EP 4 834 884 A1**
(43) Date of publication of application: **04.11.2026**
(21) Application number: 24910799.6
(22) Date of filing: 16.12.2024
(51) Int. Cl.: C07D 235/10, C07D 235/08, C07D 235/18, C07D 403/04, A61K 31/4184, A61P 17/00, A61P 17/04

(54) **TRPV3 INHIBITOR, AND PREPARATION THEREFOR AND USE THEREOF**

(30) Priority: 29.12.2023 CN 202311849606
(71) Applicant: Hangzhou Yirui Pharmaceutical Technology Co., Ltd., Hangzhou, Zhejiang 310018 (CN)
(72) Inventor: WANG, Xiaolu, Hangzhou, Zhejiang 310018 (CN); JI, Jianfeng, Hangzhou, Zhejiang 310018 (CN); CHENG, Gang, Hangzhou, Zhejiang 310018 (CN); YE, Qing, Hangzhou, Zhejiang 310018 (CN); WANG, Chendong, Hangzhou, Zhejiang 310018 (CN); LI, Yingying, Hangzhou, Zhejiang 310018 (CN)
(74) Representative: Abel & Imray LLP
(86) International application number: PCT/CN2024/139494
(87) International publication number: WO 2025/139868

(57) **Abstract**

Provided in the present invention are a compound as shown in General Formula (I) as a TRPV3 inhibitor or a pharmaceutically acceptable salt thereof and a preparation method therefor. The compound or the pharmaceutically acceptable salt thereof can be used for treating or preventing TRPV3-related skin diseases. Further provided in the present invention are a pharmaceutical composition comprising the compound or the pharmaceutically acceptable salt thereof, and the use of the compound or the pharmaceutically acceptable salt thereof in the treatment of TRPV3-related skin diseases.

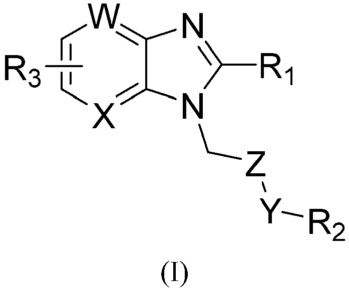

## Description

### Field of the Invention

The present invention relates to compounds as TRPV3 inhibitors and a preparation method thereof, a pharmaceutical composition comprising such compounds, as well as use of such compounds in the treatment of diseases. Specifically, the present invention relates to aromatic ring-fused imidazole-based derivatives that may be useful as TRPV3 inhibitors and a preparation method thereof, a pharmaceutical composition comprising such derivatives, as well as use of the aromatic ring-fused imidazole-based derivatives in the treatment of a TRPV3-related skin diseases.

### Background of the Invention

Transient receptor potential (TRP) channels as a family of non-selective cation channels were first discovered in Drosophila photoreceptor cells by Cosen et al. in 1969. There are 28 members of mammalian TRP channels, which are divided into 7 subgroups based on amino acid sequence homology: TRPC (Canonical), TRPV (Vanilloid), TRPM (Melastatin), TRPP (Polycystin), TRPA (Ankyrin), TRPML (Mucolipin), and TRPN (NOMPC-like). These channels are activated through a variety of mechanisms and are involved in nearly all sensory modalities.

The TRPV subfamily of transient receptor potential channels consists of 6 members: TRPV1 to TRPV6. All the 6 TRPV subfamily members possess an ankyrin repeated domain at their N-terminals. The 33 residues that make up each ankyrin repeat sequence typically serve as an important motif for subunit-subunit interactions. Among the 6 subfamily members, TRPV1, TRPV2, TRPV3, and TRPV4 are temperature-sensitive channels with suitable permeability for Ca²⁺, and TRPV5 and TRPV6 are highly selective channels for Ca²⁺. As a temperature-sensitive channel with suitable permeability for Ca²⁺, TRPV3 shares high homology with TRPV2, TRPV1, and TRPV4: 42% homology with TRPV1, 43% homology with TRPV2, and 41% homology with TRPV4.

Structurally, TRPV3 has cytoplasmic amino-terminal and carboxyl-terminal, an ankyrin repeat sequence, an amino-terminal helix-helix domain, 6 transmembrane segments, one reentrant pore loop, and many potential phosphorylation sites. Compared to the ankyrin repeat domains of TRPV1 and TRPV4, TRPV3 is believed to have 6 ankyrin repeat sequences, with one insertion in repeat 1 and 2 transient deletions in repeats 4 and 5. Moreover, TRPV3 has a unique bent Finger 3 Loop, which is maintained through hydrogen bonds and hydrophobic stacking.

TRPV3 is expressed most abundantly in human and mouse skin keratinocytes and oral and nasal epithelia, while also expressed in a variety of cells, such as the brain, spinal cord, DRG, TG, and testes. TRPV1 is expressed in sensory neurons. More specifically, TRPV1 is expressed in approximately half of somatic cells and visceral sensory neurons, with expression restricted to small to medium-sized neurons in the dorsal root ganglion, trigeminal ganglion, and vagal ganglion. In addition, TRPV1 is also expressed in perivascular sensory neurons to regulate vasodilation. TRPV1 expressed in arteriolar smooth muscle may control blood flow to skeletal muscle and certain thermoregulatory tissues, such as the skin, trachea, and cremaster muscle. TRPV2 is highly expressed in the brain, lung, and spleen, and additionaly is also expressed in endocrine cells, epithelial cells, immune cells, and cardiomyocytes. Furthermore, TRPV2 is expressed in multiple regions of the brain. TRPV4 is widely expressed in numerous tissues.

Compared to TRPV1, the most studied member of the TRPV family, TRPV3 has been developed slowly due to a lack of effective and selective agonists or inhibitors. The analysis of TRPV3 structure with the cryo-electron microscopy provides new opportunities for TRPV3 research. However, most initial TRPV3 inhibitors were natural products, requiring high effective doses and potentially causing serious side effects. Subsequently, three companies, Glenmark Pharmaceuticals, Hydra Biosciences, and Abbvie Inc., began developing small molecule TRPV3 inhibitors, but the existing TRPV3 inhibitors still suffer from issues such as insufficient efficacy and poor selectivity.

Therefore, there is still a need for effective TRPV3 inhibitors, especially specific TRPV3 inhibitors that are highly selective over other TRP channels such as TRPV1, TRPV4 and TRPA1.

### Summary of the Invention

In a first aspect, the present invention provides a small molecule inhibitor of TRPV3, which is a compound represented by a General Formula (I) or a pharmaceutically acceptable salt thereof, 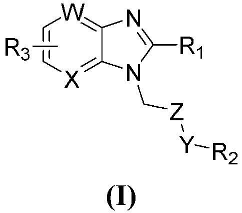 wherein:
R₁ may be selected from the group consisting of hydrogen, hydroxyl, - C(=O)-NR'₂, -S(=O)₂-R', -S(=O)₂-NR'₂, C₁-C₆ alkyl, halogenated C₁₋₆ alkyl, C₁-C₆ alkoxy, phenyl, benzyl, 5-6 membered heteroaryl, C₃-C₇ cycloalkyl, C₃-C₇ heterocyclyl and C₉-C₁₀ fused bicyclic heteroaryl, wherein R' is independently H or C₁-C₃ alkyl, the 5-6 membered heteroaryl, C₃-C₇ heterocyclyl and C₉-C₁₀ fused bicyclic heteroaryl may contain 1 or 2 heteroatoms independently selected from the group consisting of N, O and S and may be optionally oxo-substituted, and R₁ may be optionally substituted by one or more groups independently selected from the group consisting of hydroxyl, halogen, amino, cyano, nitro, C₁-C₃ alkyl, C₁-C₃ alkoxy, halogenated C₁-C₃ alkyl, C₁-C₃ fluoroalkoxy, C₁-C₃ alkylamino, di C₁-C₃ alkylamino, acetylamino, N-methyl-N-acetylamino and 5-6 membered cycloamino;
R₂ may be selected from the group consisting of phenyl, 5-6 membered heteroaryl, C₃-C₇ cycloalkyl, C₃-C₇ heterocyclyl, wherein the 5-6 membered heteroaryl and C₃-C₇ heterocyclyl may contain 1 or 2 heteroatoms independently selected from the group consisting of N, O and S and may be optionally oxo-substituted, and R₂ may be optionally substituted by one or more substituents independently selected from the group consisting of hydroxyl, halogen, amino, cyano, nitro, C₁-C₃ alkyl, C₁-C₃ alkoxy, halogenated C₁-C₃ alkyl, C₁-C₃ fluoroalkoxy, C₁-C₃ alkylamino, di C₁-C₃ alkylamino, acetylamino, N-methyl-N-acetylamino or 5-6 membered cycloamino;
R₃ may be selected from the group consisting of hydrogen, C₁₋₆ alkyl, halogenated C₁₋₆ alkyl, C₁₋₆ alkoxy, hydroxyl, halogen, cyano, nitro, amino, -S(=O)₂-R' and -S(=O)₂-NR'₂, wherein R' is independently H or C₁-C₃ alkyl;
W and X are each independently selected from the group consisting of N and C;
Z may be selected from the group consisting of -CH₂-, -CH₂-CH₂- and -C(=O)-, and Z may be optionally substituted by one or more groups independently selected from the group consisting of hydroxyl, halogen, amino, cyano, nitro, C₁-C₃ alkyl, C₁-C₃ alkoxy, halogenated C₁-C₃ alkyl, C₁-C₃ fluoroalkoxy, C₁-C₃ alkylamino, di C₁-C₃ alkylamino, acetylamino, N-methyl-N-acetylamino or 5-6 membered cycloamino; and
Y may be selected from the group consisting of -O-, -S-, -NH- and - N(C₁-C₃ alkyl)-.

In one embodiment, in the General Formula (I), R₁ may be selected from the group consisting of hydrogen, hydroxyl, -S(=O)₂-R', -S(=O)₂-NR'₂, phenyl, benzyl, pyridyl, benzothiazolyl, C₁₋₃ alkyl, C₃₋₇ cycloalkyl, halogenated C₁₋₃ alkyl and C₁₋₃ alkoxy, wherein R' is independently H or C₁-C₃ alkyl, and R₁ may be optionally substituted by one or more substituents independently selected from the group consisting of hydroxyl, halogen, amino, cyano, nitro, C₁-C₃ alkyl, C₁-C₃ alkoxy, halogenated C₁-C₃ alkyl, C₁-C₃ fluoroalkoxy and C₁-C₃ alkylamino.

In the embodiment, R₂ may be selected from the group consisting of phenyl and 5-6 membered heteroaryl, wherein the 5-6 membered heteroaryl may contain 1 or 2 heteroatoms independently selected from the group consisting of N, O and S and is optionally oxo-substituted, and R₂ may be optionally substituted by one or more substituents independently selected from the group consisting of hydroxyl, halogen, amino, cyano, nitro, C₁-C₃ alkyl, C₁-C₃ alkoxy, halogenated C₁-C₃ alkyl, C₁-C₃ fluoroalkoxy and C₁-C₃ alkylamino.

Preferably, R₂ may be selected from the group consisting of phenyl and 5-6 membered heteroaryl containing 1-2 heteroatoms selected from the group consisting of O, N and S, and R₂ may be optionally substituted by 1-3 substituents independently selected from the group consisting of halogen, cyano, C₁₋₃ alkyl, halogenated C₁₋₃ alkyl and C₁₋₃ alkoxy.

In the embodiment, R₃ may be selected from the group consisting of hydrogen, halogen, cyano, nitro, amino, hydroxyl, -S(=O)₂-R', -S(=O)₂-NR'₂, C₁₋₃ alkyl, halogenated C₁₋₃ alkyl, C₁₋₃ alkoxy, wherein R' is independently H or C₁-C₃ alkyl.

In the embodiment, W and X are each independently selected from the group consisting of N and C.

In the embodiment, Z may be selected from the group consisting of - CH₂-, -CH₂-CH₂-, -C(=O)- and -CH(OH)CH₂-.

In the embodiment, Y may be selected from the group consisting of -O-, -S- and -NH-.

In another embodiment, in the General Formula (I), R₁ may be selected from the group consisting of hydrogen, C₁-C₆ alkyl, halogenated C₁-C₆ alkyl, phenyl, benzyl, 5-6 membered heteroaryl, C₃-C₇ cycloalkyl and C₉-C₁₀ fused bicyclic heteroaryl, the 5-6 membered heteroaryl, C₃-C₇ heterocyclyl and C₉-C₁₀ fused bicyclic heteroaryl contain 1 or 2 heteroatoms independently selected from the group consisting of N, O and S, and R₁ may be optionally substituted by one or more groups independently selected from the group consisting of hydroxyl, halogen, amino, cyano, and nitro.

Preferably, R₁ may be selected from the group consisting of halogenated C₁-C₆ alkyl, phenyl, benzyl, 5-6 membered heteroaryl, C₃-C₇ cycloalkyl and C₉-C₁₀ fused bicyclic heteroaryl, the 5-6 membered heteroaryl, C₃-C₇ heterocyclyl and C₉-C₁₀ fused bicyclic heteroaryl may contain 1 or 2 heteroatoms independently selected from the group consisting of N, O and S, and R₁ may be optionally substituted by cyano. More preferably, R₁ may be selected from the group consisting of trifluoromethyl, phenyl, benzyl, pyridyl, cyclopentyl and benzothiazolyl, and R₁ may be optionally substituted by cyano. Further more preferably, R₁ may be trifluoromethyl.

In the embodiment, R₂ may be selected from the group consisting of phenyl and 5-6 membered heteroaryl containing 1 or 2 heteroatoms independently selected from the group consisting of N, O and S, and R₂ may be optionally substituted by one or more substituents independently selected from the group consisting of hydroxyl, halogen, amino, C₁-C₃ alkylamino, di C₁-C₃ alkylamino, cyano, nitro, C₁-C₃ alkyl, C₁-C₃ alkoxy, and halogenated C₁-C₃ alkyl. Preferably, R₂ may be selected from the group consisting of phenyl, pyridyl and pyrimidyl, and R₂ may be optionally substituted by one or more substituents independently selected from the group consisting of halogen, cyano, C₁-C₃ alkyl, C₁-C₃ alkoxy, and halogenated C₁-C₃ alkyl. More preferably, R₂ may be optionally substituted by one or more substituents independently selected from the group consisting of C₁-C₃ alkyl and halogenated C₁-C₃ alkyl. Further more preferably, R₂ may be optionally substituted by methyl or trifluoromethyl.

In the embodiment, R₃ may be selected from the group consisting of hydrogen, C₁₋₆ alkyl, halogenated C₁₋₆ alkyl, C₁₋₆ alkoxy, hydroxyl, halogen, cyano, nitro, amino, -S(=O)₂-R' and -S(=O)₂-NR'₂, wherein R' is independently H or C₁-C₃ alkyl. Preferably, R₃ may be selected from the group consisting of hydrogen, C₁₋₆ alkyl, halogenated C₁₋₆ alkyl, C₁₋₆ alkoxy, hydroxyl, halogen, cyano, -S(=O)₂-C₁-C₃ alkyl and -S(=O)₂-NH₂. More preferably, R₃ may be selected from the group consisting of C₁₋₆ alkyl, halogenated C₁₋₆ alkyl, halogen and cyano. Further more preferably, R₃ may be selected from the group consisting of C₁₋₃ alkyl, fluoro C₁₋₃ alkyl, halogen and cyano.

In the embodiment, W and X are each independently selected from the group consisting of N and C.

In the embodiment, Z may be selected from the group consisting of - CH₂-, -CH₂-CH₂- and -C(=O)-, and Z may be optionally substituted by one or more hydroxyls. Preferably, Z may be selected from the group consisting of -CH₂-, -CH₂-CH₂-, -CH(OH)-CH₂- and -C(=O)-. More preferably, Z may be -CH₂-.

In the embodiment, Y may be selected from the group consisting of -O-, -S- and -NH-. Preferably, Y may be selected from the group consisting of -O- and -NH-. More preferably, Y may be -O-.

In another embodiment, in the General Formula (I), R₁ may be halogenated C₁-C₆ alkyl. Preferably, R₁ may be fluoro C₁-C₃ alkyl. More preferably, R₁ may be trifluoromethyl.

In the embodiment, R₂ may be selected from the group consisting of phenyl and pyridyl, and R₂ may be optionally substituted by one or more substituents independently selected from the group consisting of C₁-C₃ alkyl and halogenated C₁-C₃ alkyl. Preferably, R₂ may be optionally substituted by one or more substituents independently selected from the group consisting of methyl and trifluoromethyl.

In the embodiment, R₃ may be selected from the group consisting of C₁₋₆ alkyl, halogenated C₁₋₆ alkyl, halogen and cyano. Preferably, R₃ may be selected from the group consisting of C₁₋₆ alkyl, halogenated C₁₋₆ alkyl and halogen. More preferably, R₃ may be selected from the group consisting of C₁₋₃ alkyl, fluoro C₁₋₃ alkyl and halogen. Each of W and X is C.

In the embodiment, Z may be -CH₂-.

In the embodiment, Y may be -O-.

In preferred embodiments, the compound represented by General Formula (I) may be selected from the group consisting of the following compounds:

| No. | Chemical name | Structural formula |
|---|---|---|
| **Compound 1** | 1-(2-(*p*-tolyloxy)ethyl)-2-(trifluoromethyl)-1*H-*benzo[*d*]imidazole | 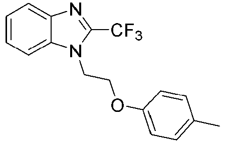 |
| **Compound 2** | 1-(2-(*p*-chlorophenoxy)ethyl)-2-(trifluoromethyl)-1*H-*benzo[*d*]imidazole | 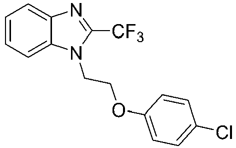 |
| **Compound 3** | 4-(2-(2-(trifluoromethyl)-1*H-*benzo[*d*]imidazol-1-yl)ethoxy)benzonitrile | 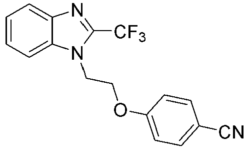 |
| **Compound 4** | 1-(2-(*o*-methoxyphenoxy)ethyl)-2-(trifluoromethyl)-1*H-*benzo[*d*]imidazole | 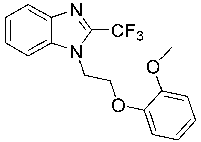 |
| **Compound 5** | 1-(3-(*p*-tolyloxy)propyl)-2-(trifluoromethyl)-1*H-*benzo[*d*]imidazole | 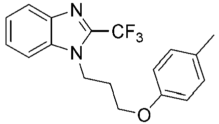 |
| **Compound 6** | *p*-methylphenyl 2-(2-(trifluoromethyl)-1*H-*benzo[*d*]imidazolyl)acetate | 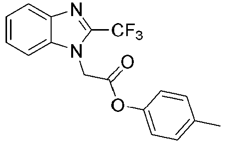 |
| **Compound 7** | 1-(*p*-tolyloxy)-3-(2-(trifluoromethyl)-1*H-*benzo[*d*]imidazolyl)2-propanol | 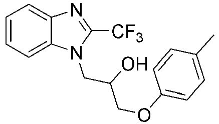 |
| **Compound 8** | 5,6-difluoro-1-(2-(*p-*tolyloxy)ethyl)-2-(trifluoromethyl)-1*H-*benzo[*d*]imidazole | 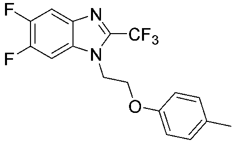 |
| **Compound 9** | 5,6-dichloro-1-(2-(*p-*tolyloxy)ethyl)-2-(trifluoromethyl)-1*H-*benzo[*d*]imidazole | 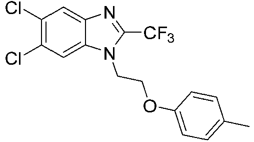 |
| **Compound 10** | 4-methyl-*N*-(2-(2-(trifluoromethyl)-1*H-*benzo[*d*]imidazol-1-yl)ethyl)aniline | 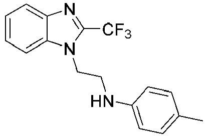 |
| **Compound 11** | 2-(benzyl)-5,6-difluoro-1-(2-(*p-*tolyloxy)ethyl)-1*H-*benzo[*d*]imidazole | 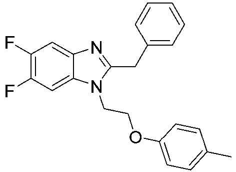 |
| **Compound 12** | 2-cyclopentyl-5,6-difluoro-1-(2-(*p*-tolyloxy)ethyl)-1*H-*benzo[*d*]imidazole | 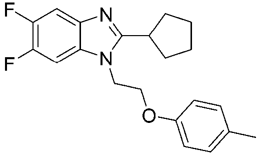 |
| **Compound 13** | 2-(phenyl)-5-fluoro-1-(2-(*p-*tolyloxy)ethyl)-1*H-*benzo[*d*]imidazole | 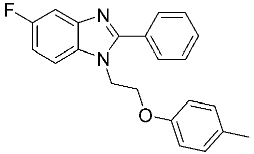 |
| **Compound 14** | 2-(5,6-difluoro-1-(2-(*p-*tolyloxy)ethyl)-1*H-*benzo[*d*]imidazol-2-yl)benzothiazole | 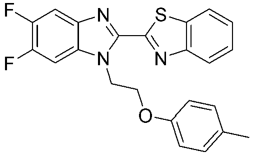 |
| **Compound 15** | 6-(5-fluoro-1-(2-(*p-*tolyloxy)ethyl)-1*H-*benzo[*d*]imidazol-2-yl)nicotinonitrile | 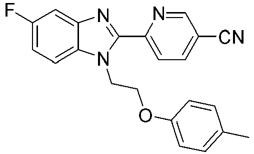 |
| **Compound 16** | 1-(2-(*p*-tolyloxy)ethyl)-2,5-bis-(trifluoromethyl)-1*H-*benzo[*d*]imidazole | 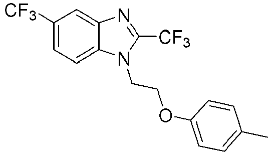 |
| **Compound 17** | 5-fluoro-1-(2-(*p*-tolyloxy)ethyl)-2-(trifluoromethyl)-1*H-*benzo[*d*]imidazole | 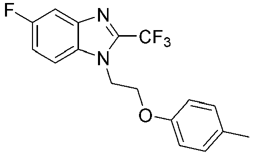 |
| **Compound 18** | 1-(2-((5-methylpyrid-2-yl)oxy)ethyl)-2,5-bis(trifluoromethyl)-1*H-*benzo[*d*]imidazole | 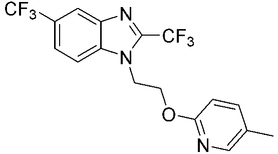 |
| **Compound 19** | 6-chloro-3-(2-(*p*-tolyloxy)ethyl)-2-(trifluoromethyl)-3*H-*imidazo[4,5-*b*]pyridine | 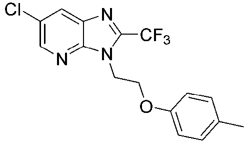 |
| **Compound 20** | 6-fluoro-3-(2-(*p*-tolyloxy)ethyl)-2-(trifluoromethyl)-3*H-*imidazo[4,5-*b*]pyridine | 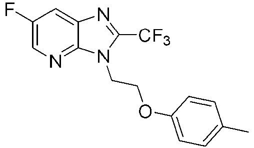 |
| **Compound 21** | 1-(2-(*p*-tolyloxy)ethyl)-2-(trifluoromethyl)-1*H-*benzo[*d*]imidazol-5-ol | 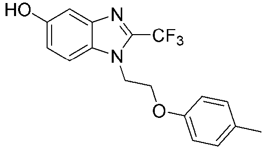 |
| **Compound 22** | 1-(2-(*p*-tolyloxy)ethyl)-2-(trifluoromethyl)-1*H-*benzo[*d*]imidazol-5-cyanide | 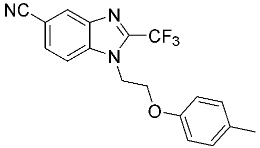 |
| **Compound 23** | 1-(2-(*p*-tolyloxy)ethyl)-2-(trifluoromethyl)-1*H-*benzo[*d*]imidazol-5-sulfonamide | 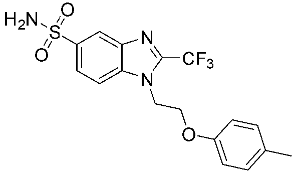 |
| **Compound 24** | 5-(methylsulfonyl)-1-(2-(*p-*tolyloxy)ethyl)-2-(trifluoromethyl)-1*H-*benzo[*d*]imidazole | 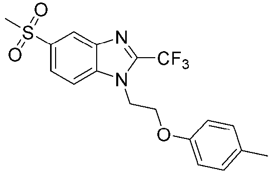 |
| **Compound 25** | 5-chloro-1-(2-(*p*-tolyloxy)ethyl)-2-(trifluoromethyl)-1*H-*benzo[*d*]imidazole | 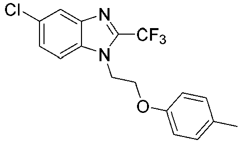 |
| **Compound 26** | 5-methyl-2-(trifluoromethyl)-1-(2-((6-(trifluoromethyl)pyrid-3-yl)oxy)ethyl)-1*H-*benzo[*d*]imidazole | 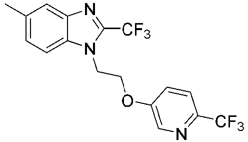 |
| **Compound 27** | 1-(2-((2-methylpyrimid-5-yl)oxy)ethyl)-2,5-bis(trifluoromethyl)-1*H-*benzo[*d*]imidazole | 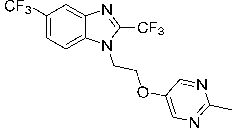 |
| **Compound 28** | 1-(2-((6-methylpyrid-3-yl)oxy)ethyl)-2,5-bis(trifluoromethyl)-1*H-*benzo[*d*]imidazole | 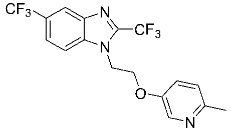 |
| **Compound 29** | 1-(2-(pyrid-4-yloxy)ethyl)-2,5-bis(trifluoromethyl)-1*H-*benzo[*d*]imidazole | 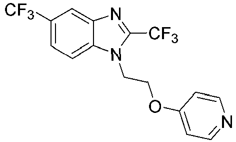 |
| **Compound 30** | 5-methyl-2-(trifluoromethyl)-1-(2-(4-(trifluoromethyl)phenoxy)ethyl)-1*H-*benzo[*d*]imidazole | 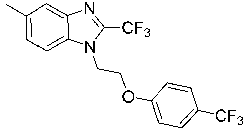 |
| **Compound 31** | *N*-(2-(5-fluoro-2-(trifluoromethyl)-1*H-*benzo[*d*]imidazol-1-yl)ethyl)-4-methylaniline | 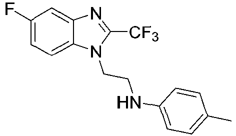 |
| **Compound 32** | *N*-(2-(5,6-difluoro-2-(trifluoromethyl)-1*H-*benzo[*d*]imidazol-1-yl)ethyl)-4-methylaniline | 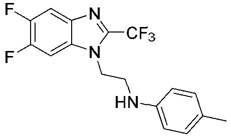 |
| **Compound 33** | *N*-(2-(5,6-dichloro-2-(trifluoromethyl)-1*H-*benzo[*d*]imidazol-1-yl)ethyl)-4-methylaniline | 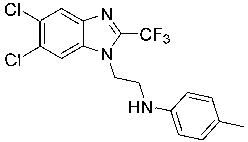 |
| **Compound 34** | *N*-(2-(2,5-bis(trifluoromethyl)-1*H-*benzo[*d*]imidazol-1-yl)ethyl)-4-methylaniline | 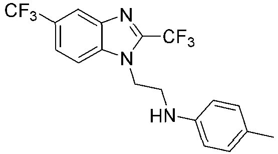 |
| **Compound 35** | *N*-(2-(5-chloro-2-(trifluoromethyl)-1*H-*benzo[*d*]imidazol-1-yl)ethyl)-4-methylaniline | 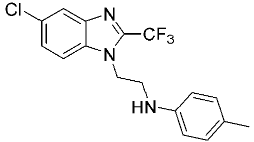 |

The compounds described herein may be prepared and/or used as a pharmaceutically acceptable salt. The pharmaceutically acceptable salt described above may be an inorganic acid salt or an organic acid salt. The inorganic acid salt described above may be a salt formed with haloid acid, nitric acid, carbonic acid, sulfuric acid, phosphoric acid, or the like. The organic acid salt described above may be a salt formed with malic acid, citric acid, fumaric acid, oxalic acid, lactic acid, camphorsulfonic acid, *p-*toluenesulfonic acid, methanesulfonic acid, benzoic acid, or the like. The haloid acid may be hydrofluoric acid, hydrobromic acid, hydroiodic acid, or hydrochloric acid. The pharmaceutically acceptable salts described above may be prepared by methods well known to those skilled in the art.

In a second aspect, the present invention provides a method for preparing the compound represented by General Formula (I) or a pharmaceutically acceptable salt thereof. The preparation method of the present invention may be achieved by one of the following schemes.

Scheme 1: A substituted 2,3-diamino aromatic ring compound represented by Formula a is condensed with an aldehyde group to obtain a compound represented by Formula b; the compound represented by Formula b is subjected to a substitution reaction with a halogenated alkane to obtain a compound represented by Formula c; the compound represented by Formula c is subjected to a substitution reaction to obtain a compound represented by General Formula (I).

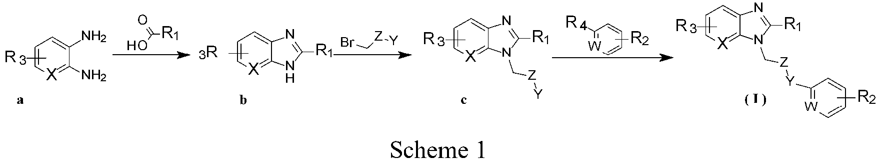

Scheme 2: A substituted 2-fluoro-1-nitro aromatic ring compound represented by Formula a is subjected to a substitution reaction with a primary amine to obtain a compound represented by Formula b'; the compound represented by Formula b' is reacted with an aromatic ring compound to obtain a compound represented by Formula c'; the compound represented by Formula c' is reduced under hydrogen to obtain a compound represented by Formula d; the compound represented by Formula d is condensed with a carboxylic acid, and then ring-closed to obtain a compound represented by General Formula (I).

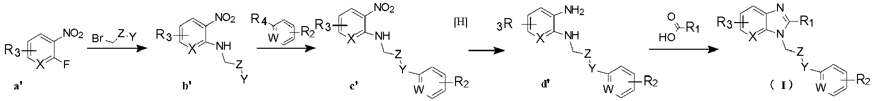

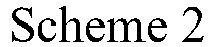

Scheme 3: A substituted 2-fluoro-1-nitro aromatic ring compound represented by Formula a" is subjected to a substitution reaction with a primary amine to obtain a compound represented by Formula b"; the compound represented by Formula b" is reduced under hydrogen to obtain a compound represented by Formula c"; the compound represented by Formula c" is condensed with a carboxylic acid, and then ring-closed to obtain a compound represented by Formula d"; the compound represented by Formula d" is reacted with an aromatic ring compound to obtain a compound presented by General Formula (I).

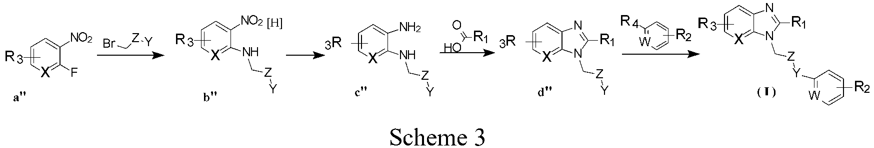

In a third aspect, the present invention provides a pharmaceutical composition comprising the compound according to the first aspect of the present invention or a pharmaceutically acceptable excipient thereof.

The pharmaceutically acceptable excipients described above may be conventional diluents, fillers, binders, wetting agents, disintegrants, absorption enhancers, surfactants, adsorption carriers, lubricants, flavoring agents, sweeteners, and the like in the pharmaceutical field.

The pharmaceutical compositions of the present invention may be formulated according to techniques known in the art. The pharmaceutical compositions may be in any form suitable for oral, parenteral, topical, intranasal, intrabronchial, sublingual, ophthalmic, otic, rectal, intravaginal, or transdermal administration. In the present invention, the pharmaceutical compositions are preferably in a form suitable for topical administration.

In a fourth aspect, the present invention provides the compound according to the first aspect of the present invention or a pharmaceutically acceptable salt thereof for use in the treatment of a TRPV3-related skin disease.

In a fifth aspect, the present invention provides a method for treating a TRPV3-related skin disease, comprising administering the compound according to the first aspect of the present invention or a pharmaceutically acceptable salt thereof to a subject in need thereof.

In a sixth aspect, the present invention provides a use of the compounds according to the first aspect of the present invention or a pharmaceutically acceptable salt thereof in the manufacture of a medicament for treating a TRPV3-related skin disease.

The medicament described above may be in various dosage forms, such as tablets, capsules, patches, ointments, emulsions, suspensions, gels, powders, granules, oral solutions, and injections, and the like. Preferably, the medicament is in a dosage form suitable for topical administration. These dosage forms may be prepared according to conventional methods in the pharmaceutical field.

In the present invention, the TRPV3-related skin disease includes, but is not limited to, chronic pruritus, acute pruritus, atopic dermatitis, psoriasis, eczema, neuropathic pain, Olmsted syndrome, hereditary palmoplantar keratoderma, and the like.

### Description of the Figures

Figure 1 shows the number of scratches within 30 minutes after administration in a carvacrol-induced pruritus model in mice;
Figure 2 shows the statistical results of the total number of scratches within 30 minutes after administration in a carvacrol-induced pruritus model in mice. *P<0.05, ****P<0.0001 compared with the normal control group; ^{△}P<0.05, ^{△△}P<0.01, ^{△△△}P<0.001 compared with the model group; ^{#}P<0.05, ^{##}P<0.01, ^{####}P<0.0001 compared with the vehicle control group.

### Detailed Description of the Invention

The present invention is further described with reference to the following examples. The following examples are intended only to illustrate the present invention and are not intended to limit the present invention in any way.

The abbreviations used in the following synthetic steps are as follows:

| | |
|---|---|
| DMF | *N*,*N*-dimethylformamide |
| K₂CO₃ | Potassium carbonate |
| PE | Petroleum ether |
| EA | Ethyl acetate |
| H₂O | Water |
| THF | Tetrahydrofuran |
| DIPEA | *N*,*N*-diisopropylethylamine |
| DEAD | Diethyl azodicarboxylate |
| DCM | Dichloromethane |
| TFA | Trifluoroacetic acid |
| NaOH | Sodium hydroxide |
| t-BuONa | Sodium tert-butoxide |
| BINAP | 2,2'-bisdiphenylphosphino-1,1'-binaphthyl |
| Pd₂(dba)₃ | Tris(dibenzylideneacetone)dipalladium |
| EtOH | Ethanol |
| Con.HCl | Concentrated hydrochloric acid |
| HATU | 2-(7-azabenzotriazole)-*N,N,N',N'*-tetramethyluronium hexafluorophosphate |
| DCE | dichloroethane |
| ACE | acetone |
| KI | Potassium iodide |
| ACN | Acetonitrile |

### Preparation Example 1. Synthesis of Compounds 1-7

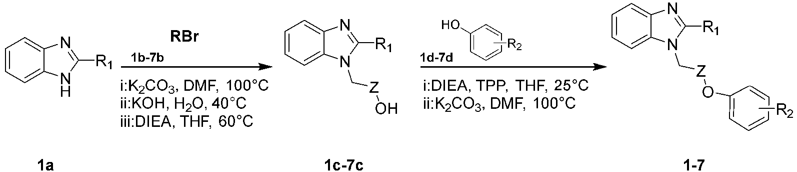

| Step 1 Reagent No. | Step 1 Reagent R-Br Structure | Step 1 Intermed iate No. | Step 1 Intermediate Structure | Step 2 Reagen t No. | Step 2 Reagent Structure |
|---|---|---|---|---|---|
| 1b | 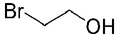 | 1c | 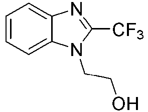 | 1d | 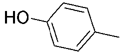 |
| 2b | 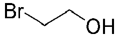 | 2c | 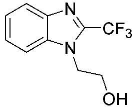 | 2d | 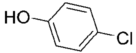 |
| 3b | 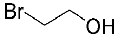 | 3c | 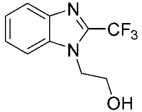 | 3d | 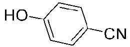 |
| 4b | 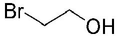 | 4c | 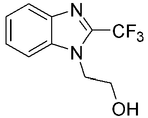 | 4d | 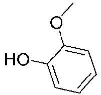 |
| 5b | 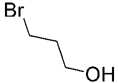 | 5c | 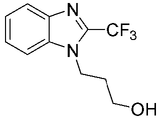 | 5d | 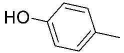 |
| 6b | 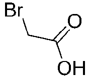 | 6c | 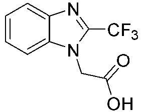 | 6d | 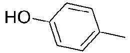 |
| 7b | 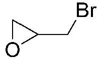 | 7c | 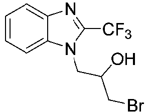 | 7d | 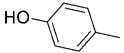 |

### Step 1. Synthesis of Intermediates 1c-7c

In a 50 mL three-necked flask, Compound **1a** (1 g, 5.4 mmol) was added and dissolved in DMF (10 mL), and Compounds **1b-5b** (10.8 mmol) and K₂CO₃ (16.2 mmol) were added respectively. The mixture was heated to 100°C and the reaction was carried out for 16 hours. After the reaction was completed, the mixture was cooled to the room temperature, diluted with water, extracted with ethyl acetate, washed with saturated brine, dried over anhydrous sodium sulfate, and concentrated to obtain a crude product. The crude product was purified by column chromatography (PE:EA=10:1) to give Compounds **1c-5c** in yields of 88%, 88%, 88%, 88%, and 86%, respectively.

In a 50 mL three-necked flask, Compound **1a** (1g, 5.4 mmol) was added and dissolved in H₂O (10 mL), and Compound **6b** (8.1 mmol) and K₂CO₃ (27 mmol) were added. The mixture was heated to 40°C and the reaction was carried out for 16 hours. After the reaction was completed, the mixture was cooled to the room temperature, diluted with water, adjusted to have a pH of 1 with 1N hydrochloric acid, extracted with ethyl acetate, dried over anhydrous sodium sulfate, and concentrated to give Compound 6c in a yield of 91%.

In a 50 mL three-necked flask, Compound **1a** (1 g, 5.4 mmol) was added and dissolved in THF (10 mL), and Compound **7b** (6.5 mmol) and DIPEA (10.8 mmol) were added. The mixture was heated to 60°C and the reaction was carried out for 16 hours. After the reaction was completed, the mixture was cooled to the room temperature, diluted with water, extracted with ethyl acetate, washed with saturated brine, dried over anhydrous sodium sulfate, and concentrated to obtain a crude product. The crude product was purified by column chromatography (PE:EA=10:1) to give Compound **7c** in a yield of 46%.

### Step 2. Synthesis of Compounds 1-7

In a 50 mL three-necked flask, Compounds **1c-6c** (4.3 mmol) were added and dissolved in THF (10 mL) respectively, and Compounds **1d-6d** (5.6 mmol) and triphenylphosphine (5.6 mmol) were added respectively. The mixture was cooled down to 0°C. DEAD (5.6 mmol) was dropwisely added. The reaction was carried out for 16 hours at 25°C. After the reaction was completed, the mixture was diluted with water, extracted with ethyl acetate, washed with saturated brine, dried over anhydrous sodium sulfate, and concentrated to give a crude product. The crude product was purified by column chromatography (PE:EA = 30:1) to give Compounds **1-6** in yields of 86%, 61%, 86%, 86%, 86%, and 86%, respectively.

In a 50 mL three-necked flask, Compound **7c** (0.8 g, 2.5 mmol) was added and dissolved in DMF (10 mL), and p-cresol (0.5 g, 5.0 mmol) and K₂CO₃ (1.0 g, 7.5 mmol) were added. The mixture was heated to 100°C and the reaction was carried out for 16 hours. After the reaction was completed, the mixture was cooled to the room temperature. The reaction solution was diluted with water, extracted once with ethyl acetate, washed with saturated brine, dried over anhydrous sodium sulfate, and concentrated to obtain a crude product. The crude product was purified by column chromatography (PE:EA=10:1) to give Compound **7** in a yield of 57%.

The structures of the synthesized Compounds **1-7** were confirmed by NMR and mass spectrometry. The ¹*H* NMR and MS (ESI) results of Compounds **1-7** are shown in the following table.

| Compound No. | Compound Name and Structure | **¹H NMR** and **MS (ESI)** |
|---|---|---|
| Compound **1** | 1-(2-(*p*-tolyloxy)ethyl)-2-(trifluoromethyl)-1*H-*benzo[*d*]imidazole 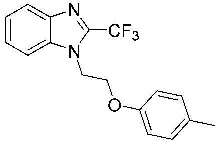 | **¹H NMR (400 MHz, CDCl₃):** δ 7.82 (dt, *J*=7.2, 2.9 Hz, 1H), 7.55-7.47 (m, 3H), 7.27 (d, *J*=8.2 Hz, 2H), 6.97 (d, *J*=8.4 Hz, 2H), 4.55 (t, *J*=6.1 Hz, 2H), 3.69 (t, *J*=6.4 Hz, 2H), 2.42 (s, 3H). |
| | | **ESI-MS:** *m*/*z*=321[M+1]⁺. |
| Compound **2** | 1-(2-(*p*-chlorophenoxy)ethyl)-2-(trifluoromethyl)-1*H-*benzo[*d*]imidazole 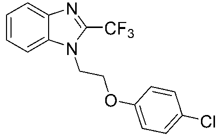 | **¹H NMR (400 MHz, CDCl₃):** δ 7.82 (dt, *J*=7.2, 2.9 Hz, 1H), 7.56-7.44 (m, 3H), 7.25 (d, *J*=8.2 Hz, 2H), 6.95 (d, *J*=8.4 Hz, 2H), 4.59 (t, *J*=6.1 Hz, 2H), 3.79 (t, *J*=6.4 Hz, 2H). |
| | | **ESI-MS:** *m*/*z*=341[M+1]⁺. |
| Compound **3** | 4-(2-(2-(trifluoromethyl)-1*H-*benzo[*d*]imidazol-1-yl)ethoxy)benzonitrile 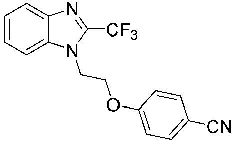 | **¹H NMR (400 MHz, CDCl₃):** δ 7.82 (dt, *J*=7.2, 2.8 Hz, 1H), 7.53-7.47 (m, 3H), 7.25 (d, *J*=8 Hz, 2H), 6.96 (d, *J*=8.4 Hz, 2H), 4.57 (t, *J*=6 Hz, 2H), 3.73 (t, *J*=6.4 Hz, 2H). |
| | | **ESI-MS:** *m*/*z*=331[M+1]⁺. |
| Compound **4** | 1-(2-(*o-*methoxyphenoxy)ethyl)-2-(trifluoromethyl)-1*H-*benzo[*d*]imidazole 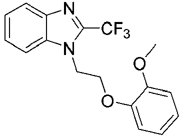 | **¹H NMR (400 MHz, CDCl₃):** δ 7.52 (dt, *J*=7.2, 2.9 Hz, 1H), 7.15-7.23 (m, 3H), 7.10 (d, *J*=8.2 Hz, 2H), 6.77 (d, *J*=8.4 Hz, 2H), 4.64 (t, *J*=6.1 Hz, 2H), 4.49 (t, *J*=6.4 Hz, 2H), 3.83 (s, 3H). |
| | | **ESI-MS:** *m*/*z*=337[M+1]⁺. |
| Compound **5** | 1-(3-(*p*-tolyloxy)propyl)-2-(trifluoromethyl)-1*H-*benzo[*d*]imidazole 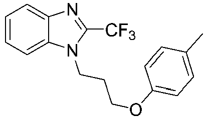 | **¹H NMR (400 MHz, CDCl₃):** δ 7.92 (dt, *J*=7.2, 2.9 Hz, 1H), 7.45-7.37 (m, 3H), 7.07 (d, *J*=8.2 Hz, 2H), 6.57 (d, *J*=8.4 Hz, 2H), 4.55 (t, *J*=6.1 Hz, 2H), 3.69 (t, *J*=6.4 Hz, 2H), 2.37 (t, *J*=6.0 Hz, 2H), 2.30 (s, 3H). |
| | | **ESI-MS:** *m*/*z*=335[M+1]⁺. |
| Compound **6** | *p*-methylphenyl 2-(2-(trifluoromethyl)-1*H-*benzo[*d*]imidazolyl)acetate 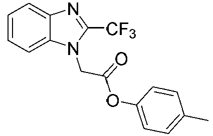 | **¹H NMR (400 MHz, CDCl₃)** δ 7.76 (dt, *J*=7.2, 2.9 Hz, 1H), 7.50-7.45 (m, 3H), 7.30 (d, *J*=8.2 Hz, 2H), 6.85 (d, *J*=8.4 Hz, 2H), 4.35 (s, 2H), 2.38 (s, 3H). |
| | | **ESI-MS:** *m*/*z*=335[M+1]⁺. |
| Compound **7** | 1-(*p*-tolyloxy)-3-(2-(trifluoromethyl)-1*H-*benzo[*d*]imidazolyl)2-propanol 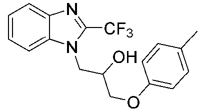 | **¹H NMR (400 MHz, CDCl₃):** δ 7.94 (dt, *J*=7.2, 2.9 Hz, 1H), 7.55-7.47 (m, 3H), 7.37 (d, *J*=8.2 Hz, 2H), 6.77 (d, *J*=8.4 Hz, 2H), 4.65 (m, 4H), 4.39 (m, 1H), 2.35 (s, 3H). |
| | | **ESI-MS:** *m*/*z*=351[M+1]⁺. |

### Preparation Example 2. Synthesis of Compounds 8-9

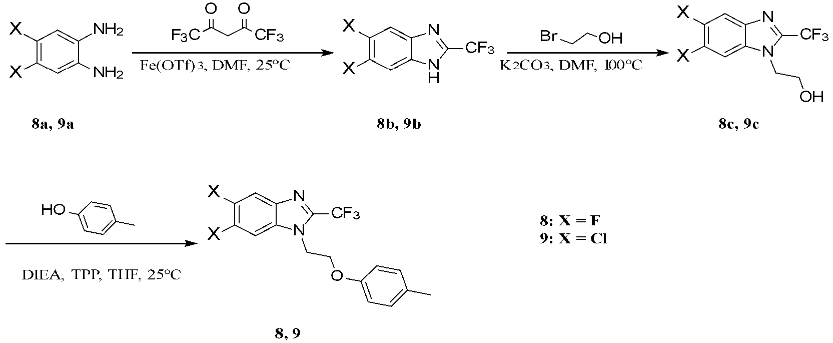

### Step 1. Synthesis of Intermediates 8b, 9b

In a 100 mL three-necked flask, Compounds **8a, 9a** (1.0 g, 6.9 mmol), hexafluoroacetylacetone (1.7 g, 8.3 mmol), iron trifluoromethanesulfonate (0.4 g, 0.7 mmol) and DMF (10 mL) were added, respectively. The mixture was heated to 80°C, and the reaction was carried out for 16 hours. After the reaction was completed, the mixture was diluted with water, extracted with ethyl acetate, washed with saturated brine, dried over anhydrous sodium sulfate, and concentrated to give a crude product. The crude product was purified by column chromatography (PE:EA = 5:1) to give Compounds **8b** and **9b** in yields of 99% and 95%, respectively.

### Step 2. Synthesis of Intermediates 8c, 9c

The synthesis steps were carried out referring to Step 1 in Preparation Example 1.

### Step 3. Synthesis of Intermediates 8, 9

The synthesis steps were carried out referring to Step 2 in Preparation Example 1.

The structures of the synthesized Compounds **8-9** were confirmed by NMR and mass spectrometry. The ¹H NMR and MS (ESI) results of Compounds **8-9** are shown in the following table.

| Compound No. | Compound Name and Structure | **¹H NMR** and **MS (ESI)** |
|---|---|---|
| Compound **8** | 5,6-difluoro-1-(2-(*p-*tolyloxy)ethyl)-2-(trifluoromethyl)-1*H-*benzo[*d*]imidazole 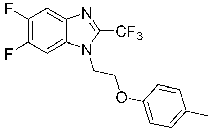 | **¹H NMR (400 MHz, CDCl₃)**:δ 7.66 (dd, *J*=9.9, 7.3 Hz, 1H), 7.55 (dd, *J*=9.8, 6.9 Hz, 1H), 7.08 (d, *J*=8.3 Hz, 2H), 6.71 (d, *J*=8.6 Hz, 2H), 4.70 (t, *J*=5.1 Hz, 2H), 4.33 (t, *J*=5.1 Hz, 2H), 2.29 (s, 3H). |
| | | **ESI-MS:** *m*/*z*=357[M+1]⁺. |
| Compound **9** | 5,6-dichloro-1-(2-(*p-*tolyloxy)ethyl)-2-(trifluoromethyl)-1*H-*benzo[*d*]imidazole 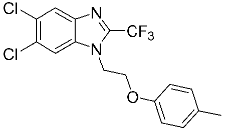 | **¹H NMR (400 MHz, CDCl₃):**δ 7.94 (s, 1H), 7.85 (s, 1H), 7.07-7.02 (m, 2H), 6.69-6.64 (m, 2H), 4.66 (t, *J*=5.1 Hz, 2H), 4.28 (t, *J*=5.1 Hz, 2H), 2.25 (s, 3H). |
| | | **ESI-MS:** *m*/*z*=389[M+1]⁺. |

### Preparation Example 3. Synthesis of Compound 10

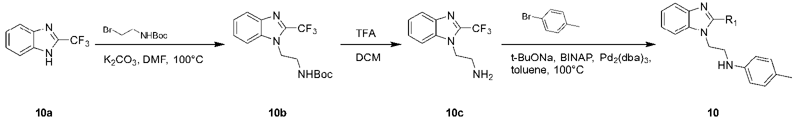

### Step 1. Synthesis of Intermediate 10b

The synthesis steps were carried out referring to Step 1 in Preparation Example 1 and using N-Boc-bromoethylamine instead of 2-bromoethanol as the starting material to synthesize Compound **10b.**

### Step 2. Synthesis of Intermediate 10c

Compound **10b** (1.0 g, 5.4 mmol) was dissolved in DCM (10 mL), and TFA (2 mL) was added. The reaction was carried out for 16 hours at 25°C. After the reaction was completed, the mixture was concentrated under reduced pressure, diluted with water, adjusted to have a pH of 9 with NaOH (1 N), extracted with ethyl acetate, washed with saturated brine, dried over anhydrous sodium sulfate, and concentrated to give Compound **10c** in a yield of 93%.

### Step 3. Synthesis of Compound 10

Compound **10c** (1.0 g, 4.4 mmol) was dissolved in toluene (10 mL), and p-bromotoluene (0.8 g, 4.4 mmol), sodium tert-butoxide (1.3 g, 13.2 mmol), BINAP (0.44 mmol) and Pd₂(dba)₃ (0.44 mmol) were added. After replacing with nitrogen, the mixture was heated to 100°C, and the reaction was carried out for 12 hours. After the reaction was completed, the mixture was cooled to the room temperature, diluted with water, extracted with ethyl acetate, washed with saturated brine, dried over anhydrous sodium sulfate, and concentrated to obtain a crude product, which was purified by column chromatography (PE:EA=10:1) to obtain a crude product. This crude product was isolated by preparative silica plate (100%DCM) to give Compound **10** in a yield of 5%.

The structure of the synthesized Compound **10** was confirmed by NMR and mass spectrometry. The ¹H NMR and MS (ESI) results of Compound **10** are shown in the following table.

| Compound No. | Compound Name and Structure | **¹H NMR** and **MS (ESI)** |
|---|---|---|
| Compound **10** | 4-methyl-*N*-(2-(2-(trifluoromethyl)-1*H-*benzo[*d*]imidal-1-yl)ethyl)aniline 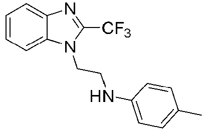 | **¹H NMR (400 MHz, CDCl₃)** δ 7.92 (dt, *J*=7.2, 2.9 Hz, 1H), 7.45-7.37 (m, 3H), 7.07 (d, *J*=8.2 Hz, 2H), 6.57 (d, *J*=8.4 Hz, 2H), 4.55 (t, *J*=6.1 Hz, 2H), 3.69 (t, *J*=6.4 Hz, 2H), 2.30(s, 3H). |
| | | **ESI-MS:** *m*/*z*=320[M+1]⁺. |

### Preparation Example 4. Synthesis of Compounds 11-15

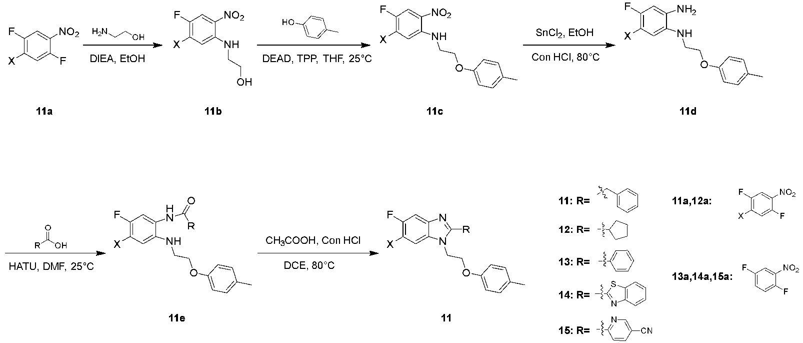

### Step 1. Synthesis of Intermediate 11b

In a 100 mL three-necked flask, Compound **11a** (4.0 g, 22.6 mmol), ethanolamine (1.5 g, 24.8 mmol), DIPEA (4.4 g, 33.9 mmol) and EtOH (40 mL) were added. The mixture was heated to 80°C and the reaction was carried out for 16 hours. After the reaction was completed, the mixture was cooled to the room temperature, concentrated under reduced pressure, diluted with water, extracted with ethyl acetate, washed with saturated brine, dried over anhydrous sodium sulfate, and concentrated to obtain a crude product. The crude product was purified by column chromatography (PE:EA= 10:1) to give Compound **11b** in a yield of 83%.

### Step 2. Synthesis of Intermediate 11c

The synthesis steps were carried out referring to to Step 2 in Preparation Example 1.

### Step 3. Synthesis of Intermediate 11d

In a 100 mL three-necked flask, Compound **11c** (2.0 g, 6.5 mmol), stannous chloride (3.6 g, 19.6 mmol), Con.HCl (6 mL) and EtOH (20 mL) were added. The mixture was heated to 80°C, and the reaction was carried out for 3 hours. After the reaction was completed, the mixture was cooled to the room temperature, concentrated under reduced pressure, diluted with water, extracted with ethyl acetate, washed with saturated brine, dried over anhydrous sodium sulfate, and concentrated to obtain a crude product. The crude product was purified by column chromatography (PE:EA= 5:1) to give Compound **11d** in a yield of 72%.

### Step 4. Synthesis of Intermediate 11e

In a 100 mL three-necked flask, Compound **11d** (1.3 g, 4.7 mmol), phenylacetic acid (0.8 g, 6.1 mmol), HATU (2.2 g, 7.1 mmol) and DMF (15 mL) were added. The reaction was carried out for 16 hours at 25°C. After the reaction was completed, the mixture was diluted with water, extracted with ethyl acetate, washed with saturated brine, dried over anhydrous sodium sulfate, and concentrated to obtain a crude product. The crude product was purified by column chromatography (PE:EA = 4:1) to give Compound **11e** in a yield of 70%.

### Step 5. Synthesis of Compound 11

In a 100 mL three-necked flask, Compound **11e** (1.3 g, 3.3 mmol), Con.HCl (5 mL), acetic acid (4 mL) and DCE (26 mL) were added. The mixture was heated to 110°C, and the reacton was carried out for 16 hours. After the reaction was completed, the mixture was cooled to the room temperature, concentrated under reduced pressure, diluted with water, extracted with dichloromethane, washed with saturated brine, dried over anhydrous sodium sulfate, and concentrated to obtain a crude product. The crude product was purified by column chromatography (PE : ACE = 1:1) to give Compound **11** in a yield of 8%.

### Step 6. Synthesis of Compounds 12-15

The synthesis steps were accired out referring to Steps 1-5 in Preparation Example 4, replacing 1,2,4-trifluoro-5-nitrobenzene as raw material with 1,4-difluoro-2-nitrobenzene, and replacing phenylacetic acid with cyclopentanoic acid, benzoic acid, benzothiazole-2-carboxylic acid, and 5-cyanopyridine-2-carboxylic acid, respectively.

The structures of the synthesized Compounds **11-15** were confirmed by NMR and mass spectrometry. The ¹H NMR and MS (ESI) results of Compounds **11-15** are shown in the following table.

| Compound No. | Compound Name and Structure | **¹H NMR** and **MS (ESI)** |
|---|---|---|
| Compound **11** | 2-(benzyl)-5,6-difluoro-1-(2-(*p-*tolyloxy)ethyl)-1*H-*benzo[*d*]imidazole 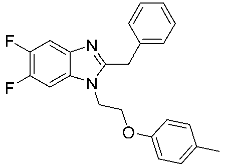 | **¹H NMR (400 MHz, CDCl₃)**:δ 7.55 (dd, *J*=10.4, 7.3 Hz, 1H), 7.39-7.19 (m, 6H), 7.06 (d, *J*=8.3 Hz, 2H), 6.63 (d, *J*=8.4 Hz, 2H), 4.43 (s, 2H), 4.36 (t, *J*=5.2 Hz, 2H), 3.96 (t, *J*=5.2 Hz, 2H), 2.29 (s, 3H). |
| | | **ESI-MS:** *m*/*z*=379[M+1]⁺. |
| Compound **12** | 2-cyclopentyl-5,6-difluoro-1-(2-(*p*-tolyloxy)ethyl)-1*H-*benzo[*d*]imidazole 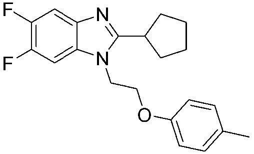 | **¹H NMR (400 MHz, CDCl₃):** δ 7.66 (dd, *J*=9.9, 7.3 Hz, 1H), 7.55 (dd, *J*=9.8, 6.9 Hz, 1H), 7.08 (d, *J*=8.3 Hz, 2H), 6.71 (d, *J*=8.6 Hz, 2H), 4.70 (t, *J*=5.1 Hz, 2H), 4.33 (t, *J*=5.1 Hz, 2H),2.71 (m, 1H), 2.29 (s, 3H). 1.93 (m, 4H).1.69 (m, 4H). |
| | | **ESI-MS:** *m*/*z*=357[M+1]⁺. |
| Compound **13** | 2-(phenyl)-5-fluoro-1-(2-(*p-*tolyloxy)ethyl)-1*H-*benzo[*d*]imidazole 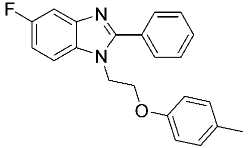 | **¹H NMR (400 MHz, CDCl₃)** δ 8.03 (s, 2H), 7.81 (d, *J*=7.1 Hz, 2H), 7.65 (s, 4H), 7.05 (d, *J*=7.2 Hz, 2H), 6.63 (d, *J*=7.8 Hz, 2H), 4.86 (s, 2H), 4.38 (s, 2H), 2.28 (s, 3H). |
| | | **ESI-MS:** *m*/*z*=347[M+1]⁺. |
| Compound **14** | 2-(5,6-difluoro-1-(2-(*p-*tolyloxy)ethyl)-1*H-*benzo[*d*]imidazol-2-yl)benzothiazole 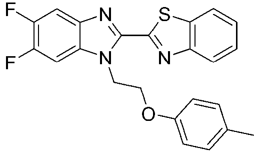 | **¹H NMR (400 MHz, CDCl₃):** δ 8.13 (d, *J*=8.0 Hz, 1H), 8.02 (d, *J*=7.9 Hz, 1H), 7.69-7.49 (m, 4H), 7.05 (d, *J*=8.3 Hz, 2H), 6.74 (d, *J*=8.6 Hz, 2H), 5.30 (t, *J*=5.1 Hz, 2H), 4.53 (t, *J*=5.1 Hz, 2H), 2.27 (s, 3H). |
| | | **ESI-MS:** *m*/*z*=422[M+1]⁺. |
| Compound **15** | 6-(5-fluoro-1-(2-(*p-*tolyloxy)ethyl)-1*H-*benzo[*d*]imidazol-2-yl)nicotinonitrile 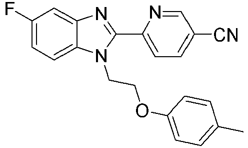 | **¹H NMR (400 MHz, CDCl₃)** δ 8.91 (s, 1H), 8.63 (d, *J*=8.4 Hz, 1H), 8.13 (dd, *J*=8.3, 2.0 Hz, 1H), 7.65-7.49 (m, 2H), 7.21 (td, *J*=9.2, 2.4 Hz, 1H), 7.04 (d, *J*=8.3 Hz, 2H), 6.65 (d, *J*=8.5 Hz, 2H), 5.22 (t, *J*=5.4 Hz, 2H), 4.44 (t, *J*=5.4 Hz, 2H), 2.29 (s, 3H). |
| | | **ESI-MS:** *m*/*z*=373[M+1]⁺. |

### Preparation Example 5. Synthesis of Compounds 16-30

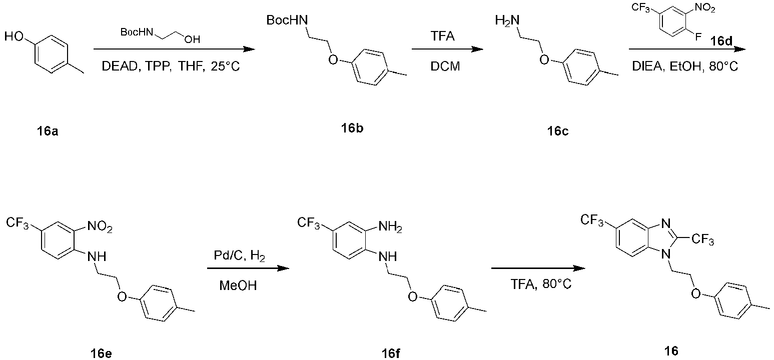

### Step 1. Synthesis of Intermediate 16b

In a 250 mL three-necked flask, Compound **16a** (5.0 g, 46.3 mmol), *N-*(*tert*-butyloxycarbonyl)ethanolamine (9.7 g, 60.2 mmol), triphenylphosphine (15.8 g, 60.2 mmol) and THF (50 mL) were added. The mixture was cooled down to 0°C. DEAD (10.5 g, 60.2 mmol) was added dropwisely. The reaction was carried out for 16 hours at 25°C. After the reaction was completed, the mixture was diluted with water, extracted with ethyl acetate, washed with saturated brine, dried over anhydrous sodium sulfate, and concentrated to obtain a crude product. The crude product was purified by column chromatography (PE:EA=5:1) to give Compound **16b** in a yield of 93%.

### Step 2. Synthesis of Intermediate 16c

Compound **16b** (11.0 g, 43.8 mmol) was dissolved in DCM (110 mL), to which TFA (15 mL) was added. The reaction was carried out for 16 hours at 25°C. After the reaction was completed, the mixture was concentrated under reduced pressure, diluted with water, adjusted to have a pH of 9 with NaOH (1N), extracted with ethyl acetate, washed with saturated brine, dried over anhydrous sodium sulfate, and concentrated to give Compound **16c** in a yield of 93%.

### Step 3. Synthesis of Intermediate 16e

In a 100 mL three-necked flask, Compound **16d** (5.0 g, 24.9 mmol), Compound **16c** (4.3 g, 28.7 mmol), DIPEA (4.8 g, 37.4 mmol) and EtOH (50 mL) were added. The mixture was heated to 80°C, and the reaction was carried out for 16 hours. After the reaction was completed, the mixture was cooled to the room temperature, concentrated under reduced pressure, diluted with water, extracted with ethyl acetate, washed with saturated brine, dried over anhydrous sodium sulfate, and concentrated to obtain a crude product. The crude product was purified by column chromatography (PE:DCM = 2:1) to give Compound **16e** in a yield of 91%.

### Step 4. Synthesis of Intermediate 16f

Compound **16e** (7.7 g, 22.6 mmol) was dissolved in methanol (70 mL), to which palladium on carbon (0.7 g, 10%) was added. The reaction was carried out at 25°C under hydrogen atmosphere for 16 hours. After the reaction was completed, the mixture was filtered to remove palladium on carbon, and concentrated under reduced pressure to give Compound **16f** in a yield of 87%.

### Step 5. Synthesis of Compound 16

Compound **16f** (6.1 g, 19.6 mmol) was dissolved in TFA (50 mL). The reaction was carried out for 16 hours at 80°C. After the reaction was completed, the mixture was concentrated under reduced pressure, diluted with water, adjusted to have a pH of 9 with NaOH (1 N), extracted with ethyl acetate, washed with saturated brine, dried over anhydrous sodium sulfate, and concentrated to obtain a crude product. The crude product was purified by column chromatography (PE:EA = 20: 1). The concentrated residue was slurried with 50 mL PE for 16 hours and then filtered. The filter cake was dried to give Compound **16** in a yield of 67%.

### Step 6. Synthesis of Compounds 17-30

The synthesis steps were carried out referring to the above Steps 1-5 in the present Preparation Example 5, replacing **16a** and **16d** as the raw material with the corresponding raw materials shown in the following table.

| Step 1 Raw Material No. | Step 1 Raw Material Structure | Step 3 Raw Material No. | Step 3 Raw Material Structure |
|---|---|---|---|
| **16a** | 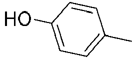 | **16d** | 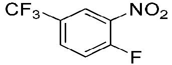 |
| **17a** | 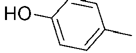 | **17d** | 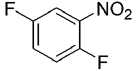 |
| **18a** | 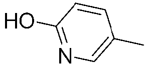 | **18d** | 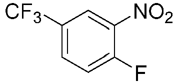 |
| **19a** | 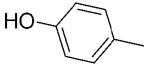 | **19d** | 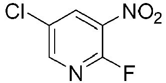 |
| **20a** | 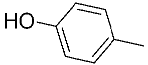 | **20d** | 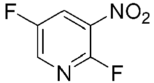 |
| **21a** | 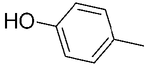 | **21d** | 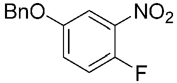 |
| **22a** | 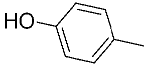 | **22d** | 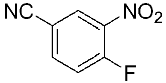 |
| **23a** | 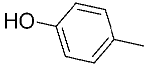 | **23d** | 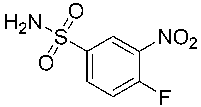 |
| **24a** | 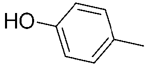 | **24d** | 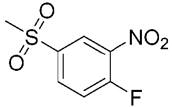 |
| **25a** | 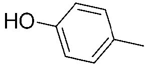 | **25d** | 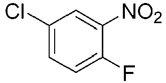 |
| **26a** | 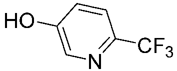 | **26d** | 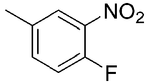 |
| **27a** | 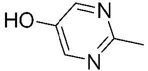 | **27d** | 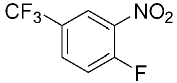 |
| **28a** | 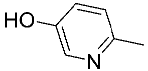 | **28d** | 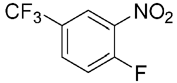 |
| **29a** | 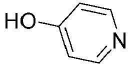 | **29d** | 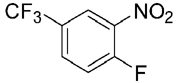 |
| **30a** | 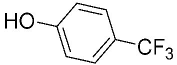 | **30d** | 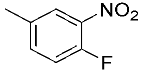 |

### Step 7. Synthesis of Intermediate 21d

In a 50 mL three-necked flask, 4-fluoro-3-nitrophenol (1.0 g, 6.4 mmol), benzyl bromide (1.2 g, 7.0 mmol), K₂CO₃ (2.0 g, 14.5 mmol), KI (1.1 g, 6.4 mmol) and ACN (10 mL) were added. The reaction was carried out for 16 hours at 25°C. The mixture was filtered, and the filtrate was spin-dried to obtain a crude product. The crude product was purified by column chromatography (PE:EA = 10:1) to obtain Intermediate **21d** in a yield of 95%.

The structures of the synthesized Compounds **16-30** were confirmed by NMR and mass spectrometry. The ¹H NMR and MS (ESI) results of Compounds **16-30** are shown in the following table.

| Compound No. | Compound Name and Structure | **¹H NMR** and **MS (ESI)** |
|---|---|---|
| Compound **16** | 1-(2-(*p*-tolyloxy)ethyl)-2,5-bis-(trifluoromethyl)-1*H-*benzo[*d*]imidazole 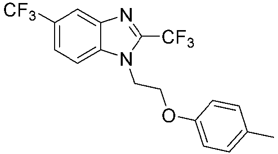 | **¹H NMR (400 MHz, CDCl₃)**:δ 8.20 (s, 1H), 7.85 (d, *J*=8.7 Hz, 1H), 7.75 (d, *J*=8.7 Hz, 1H), 7.08 (d, *J*=8.5 Hz, 2H), 6.71 (d,*J*=8.5 Hz, 2H), 4.80 (t, *J*=5.2 Hz, 2H), 4.36 (t, *J*=5.2 Hz, 2H), 2.30 (s, 3H). |
| | | **ESI-MS:** *m*/*z*=389[M+1]⁺. |
| Compound **17** | 5-fluoro-1-(2-(*p*-tolyloxy)ethyl)-2-(trifluoromethyl)-1*H-*benzo[*d*]imidazole 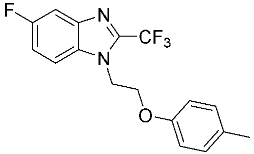 | **¹H NMR (400 MHz, CDCl₃)**:δ 7.65 (dd, *J*=9.0, 4.4 Hz, 1H), 7.56 (dd, *J*=8.9, 2.4 Hz, 1H), 7.25 (dd, *J*=9.1, 2.4 Hz, 1H), 7.07 (d,*J*=8.4 Hz, 2H), 6.71 (d, *J*=8.5 Hz, 2H), 4.74 (t, *J*=5.3 Hz, 2H), 4.34 (t, *J*=5.3 Hz, 2H), 2.29 (s, 3H). |
| | | **ESI-MS:** *m*/*z*=339[M+1]⁺. |
| Compound **18** | 1-(2-((5-methylpyrid-2-yl)oxy)ethyl)-2,5-bis(trifluoromethyl)-1*H-*benzo[*d*]imidazole 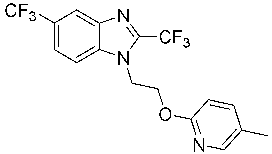 | **¹H NMR (400 MHz, CDCl₃):** δ 8.13 (dd, *J*=4.1, 3.4 Hz, 1H), 7.90-7.87 (m, 1H), 7.73-7.61(m, 2H), 7.42-7.32(m, 1H), 6.57-6.47 (m, 1H), 4.84 -4.61 (m, 4H), 2.25-2.17 (s, 3H). |
| | | **ESI-MS:** *m*/*z*=340[M+1]⁺. |
| Compound **19** | 6-chloro-3-(2-(*p-*tolyloxy)ethyl)-2-(trifluoromethyl)-3*H-*imidazo[4,5-*b*]pyridine 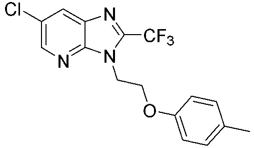 | **¹H NMR (400 MHz, CDCl₃)**:δ 8.53-8.46 (m, 1H), 8.17-8.11 (m, 1H), 7.06-6.96 (m, 2H), 6.71-6.65 (m, 2H), 4.88-4.78 (t, *J*=5.9 Hz 2H), 4.42-4.33 (t, *J*=5.9 Hz 2H), 2.27-2.19 (s, 3H). |
| | | **ESI-MS:** *m*/*z*=356[M+1]⁺. |
| Compound **20** | 6-fluoro-3-(2-(*p*-tolyloxy)ethyl)-2-(trifluoromethyl)-3*H-*imidazo[4,5-*b*]pyridine 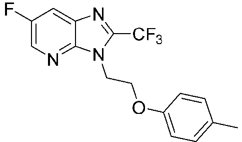 | **¹H NMR (400 MHz, CDCl₃)**:δ 8.48 (s, 1H), 7.91 (dd, *J*=8.2, 2.4 Hz, 1H), 7.06 (d, *J*=8.0 Hz, 2H), 6.78-6.68 (m, 2H), 4.89 (t, *J*=5.9 Hz 2H), 4.42 (t, *J*=5.9 Hz 2H), 2.28 (s, 3H). |
| | | **ESI-MS:** m/z=340[M+1]⁺. |
| Compound **21** | 1-(2-(*p*-tolyloxy)ethyl)-2-(trifluoromethyl)-1*H-*benzo[*d*]imidazol-5-ol 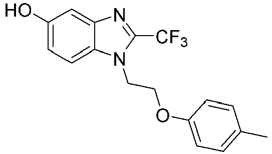 | **¹H NMR (400 MHz, CDCl₃)**:δ 7.51 (dd, *J*=8.9, 4.5 Hz, 1H), 7.29-7.25 (m, 1H), 7.08-7.00 (m, 3H), 6.71-6.65 (m, 2H), 4.68 (t,*J*=5.9 Hz 2H), 4.31-4.23 (t, *J*=5.9 Hz 2H), 2.23 (s, 3H). |
| | | **ESI-MS:** *m*/*z*=337[M+1]⁺. |
| Compound **22** | 1-(2-(*p*-tolyloxy)ethyl)-2-(trifluoromethyl)-1*H-*benzo[*d*]imidazol-5-cyanide 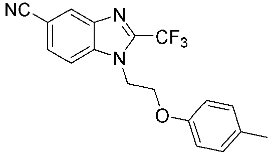 | **¹H NMR (400 MHz, CDCl₃):** δ 8.20 (dt, *J*=6.0, 3.0 Hz, 1H), 7.83-7.78 (m, 1H), 7.71-7.67 (m, 1H), 7.06-7.00 (m, 2H), 6.67-6.60 (m, 2H), 4.78-4.70 (t, *J*=5.9 Hz 2H), 4.31 (t, *J*=5.9 Hz 2H), 2.23(s, 3H). |
| | | **ESI-MS:** *m*/*z*=346[M+1]⁺. |
| Compound **23** | 1-(2-(*p*-tolyloxy)ethyl)-2-(trifluoromethyl)-1*H-*benzo[*d*]imidazol-5-sulfonamide 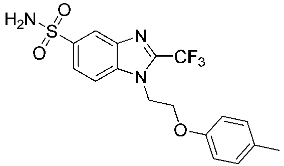 | **¹H NMR (400 MHz, CDCl₃)** δ 8.49-8.44 (m, 1H), 8.05-7.99 (m, 1H), 7.89-7.78 (m, 1H), 7.08-7.00 (m, 2H), 6.69-6.61 (m, 2H), 4.78-4.73 (t, *J*=5.9 Hz 2H), 4.38-4.25 (t, *J*=5.9 Hz 2H), 2.24 (s, 3H). |
| | | **ESI-MS:** *m*/*z*=400[M+1]⁺. |
| Compound **24** | 5-(methylsulfonyl)-1-(2-(*p-*tolyloxy)ethyl)-2-(trifluoromethyl)-1*H-*benzo[*d*]imidazole 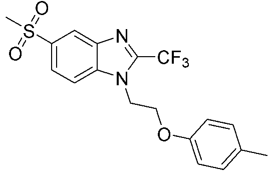 | **¹H NMR (400 MHz, CDCl₃)** δ 8.49 (dd, *J*=1.7, 0.5 Hz, 1H), 8.03 (dd, *J*=8.7, 1.7 Hz, 1H), 7.88 (dd, *J*=8.8, 0.6 Hz, 1H), 7.06-7.00 (m, 2H), 6.65 (d, *J*=8.7 Hz, 2H), 4.77 (t, *J*=5.9 Hz, 2H), 4.33 (t, *J*=5.9 Hz, 2H), 3.10 (s, 3H), 2.25 (s, 3H). |
| | | **ESI-MS:** *m*/*z*=399[M+1]⁺. |
| Compound **25** | 5-chloro-1-(2-(*p-*tolyloxy)ethyl)-2-(trifluoromethyl)-1*H-*benzo[*d*]imidazole 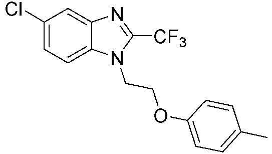 | **¹H NMR (400 MHz, CDCl₃)** δ 7.83 (t, *J*= 5.1 Hz, 1H), 7.63-7.57 (m, 1H), 7.41 (dt, *J*=15.9, 8.0 Hz, 1H), 7.06-6.98 (m, 2H), 6.73-6.61 (m, 2H), 4.69 (t, *J*=5.2 Hz, 2H), 4.28 (t, *J*=5.3 Hz, 2H), 2.24 (s, 3H). |
| | | **ESI-MS:** *m*/*z*=355[M+1]⁺. |
| Compound **26** | 5-methyl-2-(trifluoromethyl)-1-(2-((6-(trifluoromethyl)pyrid-3-yl)oxy)ethyl)-1*H-*benzo[*d*]imidazole 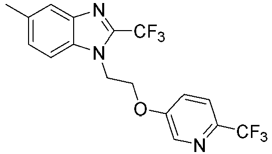 | **¹H NMR (400 MHz, CDCl₃):** δ 8.26 (d, *J*=4.0 Hz, 1H), 7.65 (s, 1H), 7.57 - 7.54 (m, 1H), 7.51 - 7.47 (m, 1H), 7.32 - 7.28 (m, 1H), 7.19 - 7.14 (m,1H), 4.76 (t, *J*=5.3 Hz, 2H), 4.45 - 4.39 (t, *J*=5.3 Hz, 2H), 2.51 - 2.47 (s, 3H). |
| | | **ESI-MS:** *m*/*z*=399[M+1]⁺. |
| Compound **27** | 1-(2-((2-methylpyrimid-5-yl)oxy)ethyl)-2,5-bis(trifluoromethyl)-1*H-*benzo[*d*]imidazole 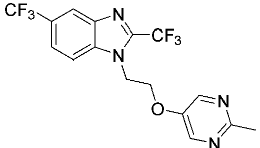 | **¹H NMR (400 MHz,CDCl₃):** δ 8.19 (s, 2H), 8.17 (s, 1H), 7.77 - 7.70 (m, 2H), 4.81 (t, *J*=5.1 Hz, 2H), 4.43 (t, *J*=5.1 Hz, 2H), 2.63 (s, 2H). |
| | | **ESI-MS:** *m*/*z*=391[M+1]⁺. |
| Compound **28** | 1-(2-((6-methylpyrid-3-yl)oxy)ethyl)-2,5-bis(trifluoromethyl)-1*H-*benzo[*d*]imidazole 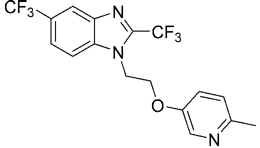 | **¹H NMR (400 MHz,CDCl₃):** δ 8.16 (dt, *J*=4.3, 2.1 Hz, 1H), 8.06 (dt, *J*= 3.4, 1.7 Hz, 1H), 7.80 - 7.68 (m, 2H), 7.05 - 6.95 (m, 2H), 4.78 (t, *J*=4 Hz, 2H), 4.37 (t, *J*=4 Hz, 2H), 2.46 (s, 3H). |
| | | **ESI-MS:** *m*/*z*=390[M+1]⁺. |
| Compound **29** | 1-(2-(pyrid-4-yloxy)ethyl)-2,5-bis(trifluoromethyl)-1*H-*benzo[*d*]imidazole 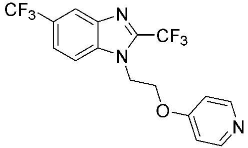 | **¹H NMR (400 MHz,CDCl₃):** δ 8.22 - 8.17 (m, 1H), 7.70 - 7.63 (m, 1H), 7.27 - 7.25 (m, 1H), 7.04 - 6.99 (m, 2H), 6.34 - 6.26 (m, 2H), 4.74 - 4.66 (m, 2H), 4.29 - 4.18 (m, 2H). |
| | | **ESI-MS:** *m*/*z*=376[M+1]⁺. |
| Compound **30** | 5-methyl-2-(trifluoromethyl)-1-(2-(4-(trifluoromethyl)phenoxy)ethyl) -1*H*-benzo[*d*]imidazole 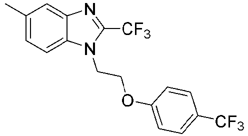 | **¹H NMR (400 MHz,CDCl₃)** δ 7.65 (d, *J*=0.7 Hz, 1H), 7.50 (dd, *J*=8.5, 4.8 Hz, 3H), 7.29 (dd, *J*=8.5, 1.3 Hz, 1H), 6.85 (d, *J*=8.6 Hz, 2H), 4.73 (t, *J*=5.4 Hz, 2H), 4.35 (t, *J*=5.4 Hz, 2H), 2.50 (s, 3H). |
| | | **ESI-MS:** *m*/*z*=389[M+1]⁺. |

### Preparation Example 6. Synthesis of Compounds 31-35

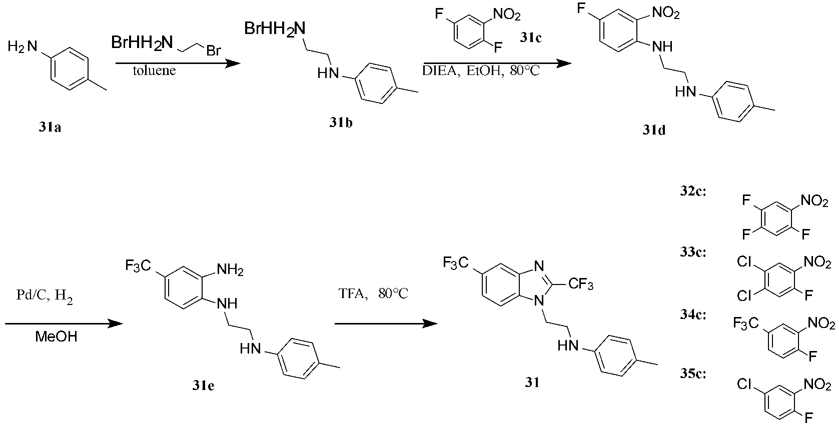

### Step 1. Synthesis of Intermediate 31b

Compound **31a** (15.7 g, 146.4 mmol) was dissolved in toluene (200 mL), to which 2-bromoethylamine hydrobromide (10.0 g, 48.8 mmol) was added. The reaction was carried out for 4 hours at 120°C. After the reaction was completed, the mixture was cooled to the room temperature. A large amount of solid was precipitated. The resultant mixture was filtered. The filter cake was washed twice with DCM (50 mL), and dried to obtain Compound **31b** in a yield of 89%.

### Step 2. Synthesis of Compounds 31-35

The synthesis steps were carried out referring to Steps 3-5 in Preparation Example 5, replacing **16c** as the raw material with **31b,** and replacing **16d** as the raw material with **31c-35c,** respectively.

The structures of the synthesized Compounds **31-35** were confirmed by NMR and mass spectrometry. The ¹H NMR and MS (ESI) results of Compounds **31-35** are shown in the following table.

| Compound No. | Compound Name and Structure | **¹H NMR** and **MS (ESI)** |
|---|---|---|
| Compound **31** | *N*-(2-(5-fluoro-2-(trifluoromethyl)-1*H-*benzo[*d*]imidazol-1-yl)ethyl)-4-methylaniline 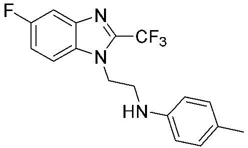 | **¹H NMR (400 MHz, CDCl₃)**:δ 7.57 (d, *J*=8.7 Hz, 1H), 7.35 (dd, *J*=8.9, 4.3 Hz, 1H), 7.18 (t, *J*=9.1 Hz, 1H), 7.10 (d, *J*=7.9 Hz, 2H), 6.65 (d, *J*=7.9 Hz, 2H), 4.58 (t, *J*=6.3 Hz, 2H), 3.69 (t, *J*=6.3 Hz, 2H), 2.32 (s, 3H). |
| | | **ESI-MS:** *m*/*z*=338[M+1]⁺. |
| Compound **32** | *N*-(2-(5,6-difluoro-2-(trifluoromethyl)-1*H-*benzo[*d*]imidazol-1-yl)ethyl)-4-methylaniline 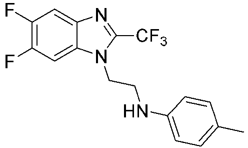 | **¹H NMR (400 MHz, CDCl₃):** δ 7.66 (dd, *J*=9.8, 7.4 Hz, 1H), 7.18 (dd, *J*=9.4, 7.0 Hz, 1H), 7.10 (d, *J*=8.0 Hz, 2H), 6.65 (d, *J*=7.9 Hz, 2H), 4.53 (t, *J*=6.2 Hz, 2H), 3.68 (t, *J*=6.3 Hz, 2H), 2.31 (s, 3H). |
| | | **ESI-MS:** *m*/*z*=356[M+1]⁺. |
| Compound **33** | *N*-(2-(5,6-dichloro-2-(trifluoromethyl)-1*H-*benzo[*d*]imidazol-1-yl)ethyl)-4-methylaniline 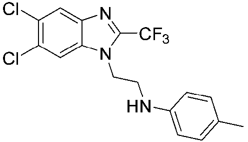 | **¹H NMR (400 MHz, CDCl₃)** δ 7.98 (d, *J*=1.6 Hz, 1H), 7.36 (d, *J*=1.7 Hz, 1H), 7.09 (d, *J*=7.6 Hz, 2H), 6.62-6.46 (m, 2H), 4.49 (t, *J*=5.9 Hz 2H), 3.69 (t, *J*=5.9 Hz 2H), 2.31 (s, 3H). |
| | | **ESI-MS:** *m*/*z*=388[M+1]⁺. |
| Compound **34** | *N*-(2-(2,5-bis(trifluoromethyl)-1*H*-benzo[*d*]imidazol-1-yl)ethyl)-4-methylaniline 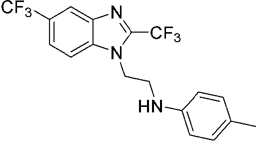 | **¹H NMR (400 MHz, CDCl₃):** δ 8.19 (s, 1H), 7.63 (d, *J*=8.6 Hz, 1H), 7.47 (d, *J*=8.7 Hz, 1H), 7.08 (d, *J*=8.1 Hz, 2H), 6.59 (d,*J*=8.3 Hz, 2H), 4.60 (t, *J*=5.9 Hz, 2H), 3.70 (t, *J*=5.9 Hz 2H), 2.30 (s, 3H). |
| | | **ESI-MS:** m/z=388 [M+1]⁺. |
| Compound **35** | *N*-(2-(5-chloro-2-(trifluoromethyl)-1*H-*benzo[*d*]imidazol-1-yl)ethyl)-4-methylaniline 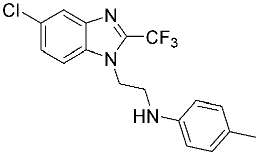 | **¹H NMR (400 MHz, CDCl₃):** δ 7.88 (d, *J*=1.5 Hz, 1H), 7.36 (dd, *J*=8.8, 1.7 Hz, 1H), 7.26 (d,*J*=8.8 Hz, 1H), 7.07 (d, *J*=8.1 Hz, 2H), 6.54 (d, *J*=8.3 Hz, 2H), 4.52 (t, *J*=5.9 Hz 2H), 3.68 (t, *J*=5.9 Hz 2H), 2.31 (s, 3H). |
| | | **ESI-MS:** *m*/*z*=354 [M+1]⁺. |

### Test Example 1. In vivo inhibitory activity evaluation test Methods and Materials

Human embryonic kidney cells (HEK-293) (obtained from the Cell Bank of the Chinese Academy of Sciences) were cultured in Dulbecco's Modified Eagle's Medium (DMEM, obtained from Gibco) supplemented with 10% fetal bovine serum (FBS, obtained from PAM) in an incubator with 95% air and 5% carbon dioxide at 37°C. The HEK-293 cells was transfected with Human TRPV3 (hTRPV3) plasmids using a calcium phosphate transfection solution comprising two types of solutions: solution A containing 250 mM CaCl₂ in pure water and solution B containing 1.5 mM Na₂HPO₄, 140 mM NaCl and 50 mM HEPES (with pH adjusted to 6.96). All the electrophysiological data were recorded 24 hours post transfection at the room temperature using an Axon 200B, with a holding potential of -60 mV. When filled with an intracellular solution containing 140 mM CsCl, 10 mM HEPES and 5 mM EGTA (with pH adjusted to 7.4 with CsOH), the patch electrode exhibited a resistance in a range of 3 to 5 MQ. The Cells were immersed in a standard extracellular solution containing 150 mM NaCl, 5 mM KCl, 10 mM glucose, 2 mM CaCl₂, 1 mM MgCl₂ and 10 mM HEPES (with pH adjusted to 7.4 with Tris-base). The TRPV3 agonist 2-APB (3 mM), the TRPV1 agonist capsaicin (1 µM), the TRPV4 agonist GSK1016790A (100 nM) as well as each of the antagonists were diluted with a Ca²⁺-free extracellular solution containing 150 mM NaCl, 5 mM KCl, 10 mM glucose and 10 mM HEPES (with pH adjusted to 7.4 with Tris-base) to prevent Ca²⁺-induced channel desensitization. The Data were collected at 10 kHz and filtered at 2 kHz. Channels were repeatedly stimulated with agonists to ensure complete channel sensitization. Each of the antagonists was pre-administered for 1 minute, and then co-administered in the presence of an agonist. In addition, the TRPA1 agonist AITC (10 µM) and its antagonist (0.01-100 µM, approximately 3-fold dilution) were diluted with an agonist solution containing 150 mM NaCl, 5 mM KCl, 1 mM MgCl₂, 10 mM glucose and 10 mM HEPES (with pH adjusted to 7.4 with Tris-base). TRPA1 was first stimulated to be openned with the agonist, followed by coadministration in the presence of the agonist. The Raw data were analyzed with a software of Origin to generate the curves. The values of IC₅₀ values calculated using the Hill equation.

### Experimental results

Representative compounds of the present invention were tested using the above described methods and materials. The values of IC₅₀ (nM) for inhibition of TRPV3 were obtained for each of the compounds. The results are shown in Table 1 below.

**Table 1. In vitro activity data of the test compounds against human TRPV3**

| Compound | Target channel | IC₅₀ (nM) |
|---|---|---|
| Compound 8 | hTRPV3 | 1100 |
| Compound 9 | hTRPV3 | 100 |
| Compound 16 | hTRPV3 | 51 |
| Compound 17 | hTRPV3 | 2440 |
| Compound 18 | hTRPV3 | 66 |
| Compound 22 | hTRPV3 | 220 |
| Compound 25 | hTRPV3 | 85 |
| Compound 30 | hTRPV3 | 98 |

From the above results, it can be seen that the compounds of the present invention can effectively inhibit TRPV3, with the values of IC₅₀ values (nM) of less than 1000 nM, preferably less than 500 nM, and more preferably less than 100 nM under the above described test conditions. Therefore, the compounds of the present invention can be used to treat TRPV3-related skin diseases, such as skin diseases caused by TRPV3 overexpression, such as pruritus and inflammation, and the like.

The values of IC₅₀ (nM) of representative compounds of the present invention for inhibition of mTRPV3 (mouse TRPV3) and other TRP channels such as hTRPV1, mTRPV4 and hTRPA1 were also determined under the same conditions using the above described methods and materials. The results are shown in Table 2 below.

**Table 2. In vitro selectivity data for the tested compounds**

| Compound | Target channel | IC₅₀ (nM) |
|---|---|---|
| | hTRPV3 | 51 |
| | mTRPV3 | 73 |
| Compound 16 | hTRPV1 | 1620 |
| | mTRPV4 | 3890 |
| | hTRPA1 | >300000 |

From the above results, it can be seen that the compounds of the present invention exhibit inhibitory activity against TRPV3 that may be several dozen times stronger than that against TRPV1, TRPV4, and TRPA1. This demonstrates that the compounds of the present invention are highly selective for TRPV3, and can act as specific and highly effective TRPV3 inhibitors.

Thus, the compounds of the present invention are able to effectively act on TRPV3 overexpression while not affecting the expression of other TRPs. Of particular note, when the compounds of the present invention are used to treat TRPV3-related skin diseases, such as pruritus and inflammation caused by TRPV3 overexpression, they are able to specifically act on TRPV3 overexpression while not affecting the expression of other TRPs, thereby avoiding side effects on the nervous system, brain and other tissues.

### Test Example 2. Pruritus Pharmacodynamics Experiment Methods and Materials

Carvacrol as a skin sensitizer has been proven to induce pruritus in mice by activating TRPV3 channels. Liu et al. found that the carvacrol-induced pruritus in mice was concentration-dependent, whereas no significant pruritus was observed in TRPV3 channel knockout mice. Therefore, the carvacrol-induced pruritus model in mice can be used to evaluate the targeting of compounds for inhibiting TRPV3 and the pharmacological efficacy against pruritus.

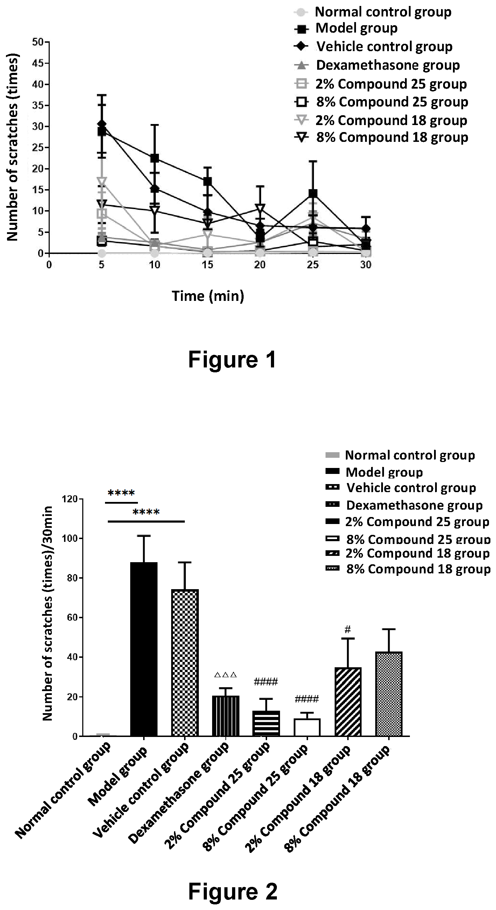

64 SPF-grade C57BL/6 mice (male, 6-8 weeks old) were used as laboratory animals. After adaptive feeding, the mice were randomly divided into 8 groups: a normal control group, a model group, a vehicle control, a positive control group, a Compound **18** (2% concentration) group, a Compound **18** (8% concentration) group, a Compound **25** (2% concentration) group and a Compound **25** (8% concentration) group, with 8 mice per group. Two days before the experiment, a 2 x 3 cm² area on the nape of the animals was depilated. 30 minutes before modeling, the respective test compound or vehicle was applied on the depilated area on the nape according to the corresponding group in an amount of 50 µl per animal. 30 minutes after administration of the test substance, all the animals except the blank control group received an intradermal injection of 2% carvacrol at the nape at an injection dose of 50 µl per animal. An acute pruritus model was established, with scratching the head with forepaws, scratching the trunk with hind paws, and biting various body parts by the mouth as pruritus indicators. Sustained scratching of the modeling area by hind paws was counted as one scratch. If the animal paused, licked its paw, or put its hind paws down during scratching, it was counted as a second scratch when scratching again. The number of scratches during the observation period was observed and recorded, with the number of scratches at each time point being counted at 5-minute intervals, and the total number of scratches within 30 minutes was summarized. The number of scratches recorded for each group within 30 minutes after drug administration is shown in Figure 1.

Raw data were analyzed using a software of GraphPad, and were statistically analyzed using one-way ANOVA. The results are shown in Figure 2. *P<0.05, ****P<0.0001 compared with the normal control group; ^{△}P<0.05, ^{△△}P<0.01, ^{△△△}P<0.001 compared with the model group; ^{#}P<0.05, ^{##}P<0.01, ^{####}P<0.0001 compared with the vehicle control group.

From the above results, it can be seen that the mice in the model group (88.00±13.35) and the mice in the vehicle control group (74.25±13.76) exhibited significant pruritus, which was statistically significantly different from the normal control group (0.75±0.41) (P<0.0001), indicating a successful model establishment. The compound dexamethasone acetate cream as the positive control, (20.63±3.84) can significantly improve the behavior of pruritus, with a significant difference compared to the model control group (P<0.001). Compound **18** at both 2% and 8% concentrations reduced pruritus numbers in mice to 34.75.00±14.71 and 42.75±11.42, respectively, in which Compound **18** at 2% concentration showed a significant difference compared to the vehicle control group (74.25±13.76) (P<0.05). Compound **25** at both 2% and 8% concentrations significantly reduced pruritus numbers in mice to 13.00±6.11 and 9.25±2.80, respectively, with a significant difference compared to the vehicle control group (74.25±13.76) (P<0.0001), and Compound **25** group also showed a lower pruritus numbers than the group of compound dexamethasone acetate cream as positive control. The results indicates that Compounds **18** and **25** can significantly improve the pruritus induced by carvacrol in mice, among which the pruritus improvement of Compound **25** tends to be better than that of dexamethasone, and exhibits a certain concentration dependence.

## Claims

1. A compound represented by a General Formula (I) or a pharmaceutically acceptable salt thereof, 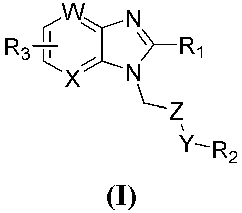 wherein:
R₁ is selected from the group consisting of hydrogen, hydroxyl, -C(=O)-NR'₂, -S(=O)₂-R', -S(=O)₂-NR'₂, C₁-C₆ alkyl, halogenated C₁₋₆ alkyl, C₁-C₆ alkoxy, phenyl, benzyl, 5-6 membered heteroaryl, C₃-C₇ cycloalkyl, C₃-C₇ heterocyclyl and C₉-C₁₀ fused bicyclic heteroaryl, wherein R' is independently H or C₁-C₃ alkyl, the 5-6 membered heteroaryl, C₃-C₇ heterocyclyl and C₉-C₁₀ fused bicyclic heteroaryl contain 1 or 2 heteroatoms independently selected from the group consisting of N, O and S and are optionally oxo-substituted, and R₁ is optionally substituted by one or more groups independently selected from the group consisting of hydroxy, halogen, amino, cyano, nitro, C₁-C₃ alkyl, C₁-C₃ alkoxy, halogenated C₁-C₃ alkyl, C₁-C₃ fluoroalkoxy, C₁-C₃ alkylamino, di C₁-C₃ alkylamino, acetylamino, N-methyl-N-acetylamino and 5-6 membered cycloamino;
R₂ is selected from the group consisting of phenyl, 5-6 membered heteroaryl, C₃-C₇ cycloalkyl, C₃-C₇ heterocyclyl, wherein the 5-6 membered heteroaryl and C₃-C₇ heterocyclyl contain 1 or 2 heteroatoms independently selected from the group consisting of N, O and S and are optionally oxo-substituted, and R₂ is optionally substituted by one or more substituents independently selected from the group consisting of hydroxyl, halogen, amino, cyano, nitro, C₁-C₃ alkyl, C₁-C₃ alkoxy, halogenated C₁-C₃ alkyl, C₁-C₃ fluoroalkoxy, C₁-C₃ alkylamino, di C₁-C₃ alkylamino, acetylamino, N-methyl-N-acetylamino or 5-6 membered cycloamino;
R₃ is selected from the group consisting of hydrogen, C₁₋₆ alkyl, halogenated C₁₋₆ alkyl, C₁₋₆ alkoxy, hydroxy, halogen, cyano, nitro, amino, - S(=O)₂-R' and -S(=O)₂-NR'₂, wherein R' is independently H or C₁-C₃ alkyl;
W and X are each independently selected from the group consisting of N and C;
Z is selected from the group consisting of -CH₂-, -CH₂-CH₂- and - C(=O)-, and Z is optionally substituted by one or more groups independently selected from the group consisting of hydroxy, halogen, amino, cyano, nitro, C₁-C₃ alkyl, C₁-C₃ alkoxy, halogenated C₁-C₃ alkyl, C₁-C₃ fluoroalkoxy, C₁-C₃ alkylamino, di C₁-C₃ alkylamino, acetylamino, N-methyl-N-acetylamino or 5-6 membered cycloamino; and
Y is selected from the group consisting of -O-, -S-, -NH- and -N(C₁-C₃ alkyl)-.

2. The compound or a pharmaceutically acceptable salt thereof according to claim 1, **characterized in that**:
R₁ is selected from the group consisting of hydrogen, hydroxyl, - S(=O)₂-R', -S(=O)₂-NR'₂, phenyl, benzyl, pyridyl, benzothiazolyl, C₁₋₃ alkyl, C₃₋₇ cycloalkyl, halogenated C₁₋₃ alkyl and C₁₋₃ alkoxy, wherein R' is independently H or C₁-C₃ alkyl, and R₁ is optionally substituted by one or more substituents independently selected from the group consisting of hydroxyl, halogen, amino, cyano, nitro, C₁-C₃ alkyl, C₁-C₃ alkoxy, halogenated C₁-C₃ alkyl, C₁-C₃ fluoroalkoxy, and C₁-C₃ alkylamino;
R₂ is selected from the group consisting of phenyl and 5-6 membered heteroaryl, wherein the 5-6 membered heteroaryl contains 1 or 2 heteroatoms independently selected from the group consisting of N, O and S and is optionally oxo-substituted, and R₂ is optionally substituted by one or more substituents independently selected from the group consisting of hydroxy, halogen, amino, cyano, nitro, C₁-C₃ alkyl, C₁-C₃ alkoxy, halogenated C₁-C₃ alkyl, C₁-C₃ fluoroalkoxy, and C₁-C₃ alkylamino; preferably, R₂ is selected from the group consisting of phenyl and 5-6 membered heteroaryl containing 1-2 heteroatoms selected from the group consisting of O, N and S, and R₂ is optionally substituted by 1-3 substituents independently selected from the group consisting of halogen, cyano, C₁₋₃ alkyl, halogenated C₁₋₃ alkyl, and C₁₋₃ alkoxy;
R₃ is selected from the group consisting of hydrogen, halogen, cyano, nitro, amino, hydroxy, -S(=O)₂-R', -S(=O)₂-NR'₂, C₁₋₃ alkyl, halogenated C₁₋₃ alkyl, and C₁₋₃ alkoxy, wherein R' is independently H or C₁-C₃ alkyl;
W and X are each independently selected from the group consisting of N and C;
Z is selected from the group consisting of-CH₂-, -CH₂-CH₂-, -C(=O)- and -CH(OH)CH₂-; and
Y is selected from the group consisting of-O-, -S- and -NH-.

3. The compound or a pharmaceutically acceptable salt thereof according to claim 1, **characterized in that**:
R₁ is selected from the group consisting of hydrogen, C₁-C₆ alkyl, halogenated C₁-C₆ alkyl, phenyl, benzyl, 5-6 membered heteroaryl, C₃-C₇ cycloalkyl and C₉-C₁₀ fused bicyclic heteroaryl, the 5-6 membered heteroaryl, C₃-C₇ heterocyclyl and C₉-C₁₀ fused bicyclic heteroaryl contain 1 or 2 heteroatoms independently selected from the group consisting of N, O and S, and R₁ is optionally substituted by one or more groups independently selected from the group consisting of hydroxyl, halogen, amino, cyano and nitro; preferably, R₁ is selected from the group consisting of halogenated C₁-C₆ alkyl, phenyl, benzyl, 5-6 membered heteroaryl, C₃-C₇ cycloalkyl and C₉-C₁₀ fused bicyclic heteroaryl, the 5-6 membered heteroaryl, C₃-C₇ heterocyclyl and C₉-C₁₀ fused bicyclic heteroaryl contain 1 or 2 heteroatoms independently selected from the group consisting of N, O and S, and R₁ is optionally substituted by cyano; more preferably, R₁ is selected from the group consisting of trifluoromethyl, phenyl, benzyl, pyridyl, cyclopentyl and benzothiazolyl, and R₁ is optionally substituted by cyano;
R₂ is selected from the group consisting of phenyl and 5-6 membered heteroaryl containing 1 or 2 heteroatoms independently selected from the group consisting of N, O and S, and R₂ is optionally substituted by one or more substituents independently selected from the group consisting of hydroxyl, halogen, amino, C₁-C₃ alkylamino, di C₁-C₃ alkylamino, cyano, nitro, C₁-C₃ alkyl, C₁-C₃ alkoxy and halogenated C₁-C₃ alkyl; preferably, R₂ is selected from the group consisting of phenyl, pyridyl and pyrimidyl, and R₂ is optionally substituted by one or more substituents independently selected from the group consisting of halogen, cyano, C₁-C₃ alkyl, C₁-C₃ alkoxy and halogenated C₁-C₃ alkyl;
R₃ is selected from the group consisting of hydrogen, C₁₋₆ alkyl, halogenated C₁₋₆ alkyl, C₁₋₆ alkoxy, hydroxyl, halogen, cyano, nitro, amino, -S(=O)₂-R' and -S(=O)₂-NR'₂, wherein R' is independently H or C₁-C₃ alkyl; preferably, R₃ is selected from the group consisting of hydrogen, C₁₋₆ alkyl, halogenated C₁₋₆ alkyl, C₁₋₆ alkoxy, hydroxyl, halogen, cyano, -S(=O)₂-C₁-C₃ alkyl and -S(=O)₂-NH₂;
W and X are each independently selected from the group consisting of N and C;
Z is selected from the group consisting of -CH₂-, -CH₂-CH₂- and - C(=O)-, and Z is optionally substituted by one or more hydroxyls; preferably, Z is selected from the group consisting of -CH₂-, -CH₂-CH₂-, -CH(OH)-CH₂- and -C(=O)-;
Y is selected from the group consisting of -O-, -S- and -NH-; preferably, Y is selected from the group consisting of -O- and -NH-.

4. The compound or a pharmaceutically acceptable salt thereof according to claim 1, **characterized in that**:
R₁ is halogenated C₁-C₆ alkyl; preferably, R₁ is trifluoromethyl;
R₂ is selected from the group consisting of phenyl and pyridyl, and R₂ is optionally substituted by one or more substituents independently selected from the group consisting of C₁-C₃ alkyl and halogenated C₁-C₃ alkyl;
R₃ is selected from the group consisting of C₁₋₆ alkyl, halogenated C₁₋₆ alkyl, halogen and cyano; preferably, R₃ is selected from the group consisting of C₁₋₆ alkyl, halogenated C₁₋₆ alkyl and halogen;
each of W and X is C;
Z is -CH₂-,
Y is -O-.

5. The compound or a pharmaceutically acceptable salt thereof according to claim 1, the compound is selected from the group consisting of:
1-(2-(*p*-tolyloxy)ethyl)-2-(trifluoromethyl)-1*H*-benzo[*d*]imidazole,
1-(2-(*p*-chlorophenoxy)ethyl)-2-(trifluoromethyl)-1*H-*benzo[*d*]imidazole,
4-(2-(2-(trifluoromethyl)-1*H*-benzo[*d*]imidazol-1-yl)ethoxy)benzonitrile,
1-(2-(*o*-methoxyphenoxy)ethyl)-2-(trifluoromethyl)-1*H-*benzo[*d*]imidazole,
1-(3-(*p*-tolyloxy)propyl)-2-(trifluoromethyl)-1*H*-benzo[*d*]imidazole,
*p*-methylphenyl 2-(2-(trifluoromethyl)-1*H*-benzo[*d*]imidazolyl)acetate,
1-(*p*-tolyloxy)-3-(2-(trifluoromethyl)-1*H*-benzo[*d*]imidazolyl)2-propanol,
5,6-difluoro-1-(2-(*p*-tolyloxy)ethyl)-2-(trifluoromethyl)-1*H-*benzo[*d*]imidazole,
5,6-dichloro-1-(2-(*p*-tolyloxy)ethyl)-2-(trifluoromethyl)-1*H-*benzo[*d*]imidazole,
4-methyl-*N*-(2-(2-(trifluoromethyl)-1*H*-benzo[*d*]imidazol-1-yl)ethyl)aniline,
2-(benzyl)-5,6-difluoro-1-(2-(*p*-tolyloxy)ethyl)-1*H*-benzo[*d*]imidazole,
2-cyclopentyl-5,6-difluoro-1-(2-(*p*-tolyloxy)ethyl)-1*H-*benzo[*d*]imidazole,
2-(phenyl)-5-fluoro-1-(2-(*p*-tolyloxy)ethyl)-1*H*-benzo[*d*]imidazole,
2-(5,6-difluoro-1-(2-(*p*-tolyloxy)ethyl)-1*H*-benzo[*d*]imidazol-2-yl)benzothiazole,
6-(5-fluoro-1-(2-(*p*-tolyloxy)ethyl)-1*H*-benzo[*d*]imidazol-2-yl)nicotinonitrile,
1-(2-(*p*-tolyloxy)ethyl)-2,5-bis-(trifluoromethyl)-1*H-*benzo[*d*]imidazole,
5-fluoro-1-(2-(*p*-tolyloxy)ethyl)-2-(trifluoromethyl)-1*H-*benzo[*d*]imidazole,
1-(2-((5-methylpyrid-2-yl)oxy)ethyl)-2,5-bis(trifluoromethyl)-1*H-*benzo[*d*]imidazole,
6-chloro-3-(2-(*p*-tolyloxy)ethyl)-2-(trifluoromethyl)-3*H*-imidazo[4,5-*b*]pyridine,
6-fluoro-3-(2-(*p*-tolyloxy)ethyl)-2-(trifluoromethyl)-3*H*-imidazo[4,5-*b*]pyridine,
1-(2-(*p*-tolyloxy)ethyl)-2-(trifluoromethyl)-1*H*-benzo[*d*]imidazol-5-ol,
1-(2-(*p*-tolyloxy)ethyl)-2-(trifluoromethyl)-1*H*-benzo[*d*]imidazol-5-cyanide,
1-(2-(*p*-tolyloxy)ethyl)-2-(trifluoromethyl)-1*H*-benzo[*d*]imidazol-5-sulfonamide,
5-(methylsulfonyl)-1-(2-(*p*-tolyloxy)ethyl)-2-(trifluoromethyl)-1*H-*benzo[*d*]imidazole,
5-chloro-1-(2-(*p*-tolyloxy)ethyl)-2-(trifluoromethyl)-1*H-*benzo[*d*]imidazole,
5-methyl-2-(trifluoromethyl)-1-(2-((6-(trifluoromethyl)pyrid-3-yl)oxy)ethyl)-1*H*-benzo[*d*]imidazole,
1-(2-((2-methylpyrimid-5-yl)oxy)ethyl)-2,5-bis(trifluoromethyl)-1*H-*benzo[*d*]imidazole,
1-(2-((6-methylpyrid-3-yl)oxy)ethyl)-2,5-bis(trifluoromethyl)-1*H-*benzo[*d*]imidazole,
1-(2-(pyrid-4-yloxy)ethyl)-2,5-bis(trifluoromethyl)-1*H-*benzo[*d*]imidazole,
5-methyl-2-(trifluoromethyl)-1-(2-(4-(trifluoromethyl)phenoxy)ethyl)-1*H*-benzo[*d*]imidazole,
*N*-(2-(5-fluoro-2-(trifluoromethyl)-1*H*-benzo[*d*]imidazol-1-yl)ethyl)-4-methylaniline,
*N*-(2-(5,6-difluoro-2-(trifluoromethyl)-1*H*-benzo[*d*]imidazol-1-yl)ethyl)-4-methylaniline,
*N*-(2-(5,6-dichloro-2-(trifluoromethyl)-1*H*-benzo[*d*]imidazol-1-yl)ethyl)-4-methylaniline,
*N*-(2-(2,5-bis(trifluoromethyl)-1*H*-benzo[*d*]imidazol-1-yl)ethyl)-4-methylaniline, and
*N*-(2-(5-chloro-2-(trifluoromethyl)-1*H*-benzo[*d*]imidazol-1-yl)ethyl)-4-methylaniline.

6. A method for preparing the compound or a pharmaceutically acceptable salt thereof according to any one of claims 1 to 5, **characterized in that**:
the method comprises the steps of:
Scheme 1: condensing substituted 2,3-diamino aromatic ring compound represented by Formula a with an aldehyde group to obtain a compound represented by Formula b; subjecting the compound represented by Formula b to a substitution reaction with a halogenated alkane to obtain a compound represented by Formula c; subjecting the compound represented by Formula c to a substitution reaction to obtain a compound represented by General Formula (I), 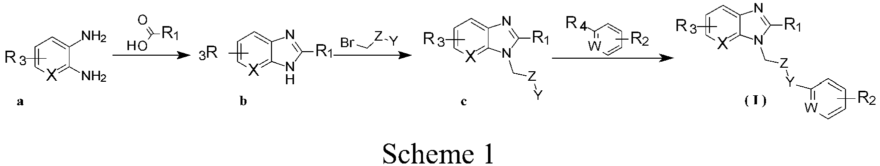
alternatively, the method comprises the steps of:
Scheme 2: subjecting a substituted 2-fluoro-1-nitro aromatic ring compound represented by Formula a to a substitution reaction with a primary amine to obtain a compound represented by Formula b'; reacting the compound represented by Formula b' with an aromatic ring compound to obtain a compound represented by Formula c'; reducing the compound represented by Formula c' under hydrogen to obtain a compound represented by Formula d; condensing the compound represented by Formula d with a carboxylic acid, and then ring-closing to obtain a compound represented by General Formula (I), 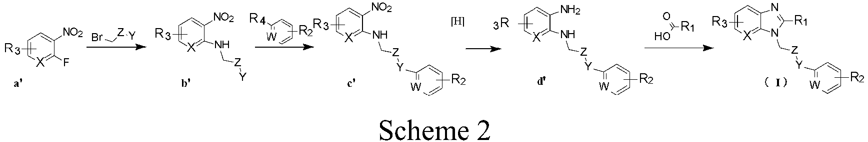
alternatively, the method comprises the steps of:
Scheme 3: subjecting a substituted 2-fluoro-1-nitro aromatic ring compound represented by Formula a" to a substitution reaction with a primary amine to obtain a compound represented by Formula b"; reducing the compound represented by Formula b" under hydrogen to obtain a compound represented by Formula c"; condensing the compound represented by Formula c" with a carboxylic acid, and then ring-closing to obtain a compound represented by Formula d"; reacting the compound represented by Formula d" with an aromatic compound to obtain a compound of General Formula (I)

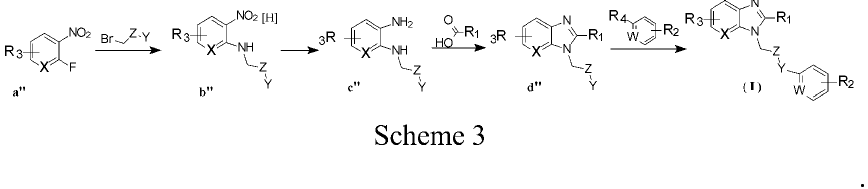

7. A pharmaceutical composition comprising the compound or a pharmaceutically acceptable salt thereof according to any one of claims 1 to 5 and one or more pharmaceutically acceptable excipients.

8. Use of the compound or a pharmaceutically acceptable salt thereof according to any one of claims 1 to 5 for treating a TRPV3-related skin disease, the skin disease is preferably selected from the group consisting of chronic pruritus, acute pruritus, atopic dermatitis, psoriasis, eczema, neuropathic pain, Olmsted syndrome, and hereditary palmoplantar keratoderma.

9. A method for treating a TRPV3-related skin disease, comprising administering an effective amount of the compound or a pharmaceutically acceptable salt thereof according to any one of claims 1 to 5 to a subject in need thereof, wherein the skin disease is preferably selected from the group consisting of chronic pruritus, acute pruritus, atopic dermatitis, psoriasis, eczema, neuropathic pain, Olmsted syndrome, and hereditary palmoplantar keratoderma.

10. Use of the compound or a pharmaceutically acceptable salt thereof according to any one of claims 1 to 5 in the manufacture of a medicament for treating a TRPV3-related skin disease, wherein the skin disease is preferably selected from the group consisting of chronic pruritus, acute pruritus, atopic dermatitis, psoriasis, eczema, neuropathic pain, Olmsted syndrome, and hereditary palmoplantar keratoderma.
